# EUROPEAN PATENT APPLICATION

(11) **EP 4 361 129 A1**
(43) Date of publication of application: **01.05.2024**
(21) Application number: 22932852.1
(22) Date of filing: 29.04.2022
(51) Int. Cl.: C07C 327/30, A61K 31/713, A61K 31/7088, A61P 31/14, A61P 11/00, A61K 47/20, A61K 9/127, A61K 39/215, A61K 39/155, A61K 39/395, A61K 39/00

(54) **CATIONIC LIPID COMPOUNDS USED FOR NUCLEIC ACID DELIVERY, COMPOSITION AND USE**

(30) Priority: 23.03.2022 CN 202210286081
(71) Applicant: Shenzhen Rhegen Biotechnology Co., Ltd., Shenzhen City, Guangdong 518057 (CN)
(72) Inventor: HU, Yong, Shenzhen, Guangdong 518057 (CN); LI, Yafei, Shenzhen, Guangdong 518057 (CN)
(74) Representative: Patentanwälte Olbricht Buchhold Keulertz
(86) International application number: PCT/CN2022/090136
(87) International publication number: WO 2023/178795

(57) **Abstract**

Provided in the present invention are cationic lipid compounds used for nucleic acid delivery, a composition and the use. The compounds are represented by the following formula (I). Further provided in the present invention are the use of nano-lipid particles using said compounds as key components in the aspect of nucleic acid delivery, a delivery-vector-containing component, a preparation method and a use method.

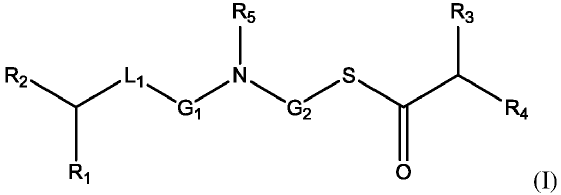

## Description

### Field of Technology

The present invention relates to the field of lipid delivery carriers. Particularly, The present invention relates to a class of cationic lipid compounds which, when combined with other lipid components, are capable of forming drug-carrying nano-lipid particles, thereby enabling the delivery of nucleic acids from outside to inside of cells *in vitro* and *in vivo.* Specifically, the present invention relates to a cationic lipid compound and a composition for delivery of nucleic acids and use thereof.

### Background Art

Nucleic acid drugs replace, compensate, block or modify specific genes by introducing exogenous genes into target cells or tissues to achieve the purpose of treating and preventing diseases. They are relatively simpler to develop and produce, and have the advantages of short R&D cycle, high success rate in clinical development, and better plasticity for improvement. Nucleic acid vaccines, as one of the mainstays of COVID-19 prevention in recent years, have also proved their great potential in the market.

However, naked mRNA has a short circulation time *in vivo,* is easily degraded, and is difficult to enter target cells or target tissues. Therefore, improving the *in vivo* delivery efficiency of mRNA drugs is one of the key directions to improve the effectiveness of this class of products.

Currently, the most widely used delivery carriers for nucleic acid drugs are lipid nanoparticles, which have the characteristics of improving the efficacy of gene drugs as well as targeted delivery effects, and can protect nucleic acids from rapid degradation *in vivo*, so as to prolong the circulation time and enhance targeted delivery. The lipid nanoparticles are comprised of 2-4 lipid components, including cationic lipid compounds, 0-2 auxiliary lipids and 0-1 PEG lipids, in which cationic lipid compounds play a key role in encapsulation and release of nucleic acids. Therefore, it is crucial to develop novel, efficient and low-toxic cationic lipid compounds.

### Summary of the Invention

The present invention provides a class of sulfur-containing cationic lipid compounds, including pharmaceutically acceptable salts thereof and stereoisomers or tautomers thereof. They are primarily used in combination with other lipid components in specific ratios, to form lipid nanoparticles for delivery of prophylactic or therapeutic agents, such as therapeutic nucleic acids.

Another object of the present invention is to provide methods for synthesizing this class of lipid compounds, by using readily available raw materials via a reaction route with mild conditions, high product yields, low instrumentation requirements and simplicity of operation.

In some examples, the therapeutic nucleic acids include plasmid DNA, messenger RNA, antisense oligonucleotides (ASON), micro RNA (miRNA), interfering RNA (micRNA), dicer substrate RNA, complementary DNA (cDNA).

The present invention also provides formulations and methods of use of such cationic lipid compounds when used in combination with other lipid components, and applications thereof in cellular and animal models.

In embodiments of the present invention, there is provided a cationic lipid compound having a structure of formula (I) as below: 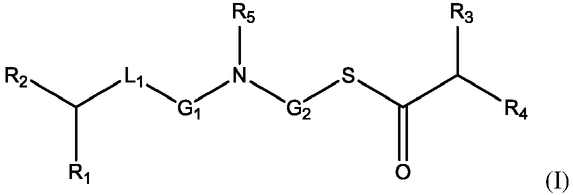
or a pharmaceutically acceptable salts, tautomers or stereoisomers thereof,
wherein:
   L₁ is -C(=O)S- or -C(=O)O-;
   G₁ and G₂ are each independently a C₃-C₁₀ alkylene;
   R₁ is a C₂-C₁₂ alkyl;
   R₂ is H or a C₂-C₁₂ alkyl;
   R₃ is a C₂-C₁₂ alkyl;
   R₄ is H or a C₂-C₁₂ alkyl;
   R₅ is H, a C₁-C₆ alkyl, R₇-OH, R₇-OC(=O)CH₃, R₇-NHC(=O)-CH₃, R₇-OCH₃ or R₇-N(R₈)₂;
   R₇ is a C₂-C₁₈ alkylene;
   R₈ is a straight or branched C₂-C₈ alkyl or forms a cyclic alkyl with N.

In some specific embodiments of the present invention, G₁ and G₂ are C₄-C₈ alkylene.

In some specific embodiments of the present invention, R₇ is a C₂-C₈ alkylene.

In some specific embodiments of the present invention, R₁ and R₃ are each independently C₄-C₈ alkyl; and R₂ and R₄ are H or C₄-C₈ alkyl.

In some specific embodiments of the present invention, one and only one of R₂ and R₄ is H.

In some specific embodiments of the present invention, R₅ is R₇-OH, and R₇ is C₂-C₈ alkylene.

In some specific embodiments of the present invention, R₅ is R₇-N(CH₂CH₃)CH₂CH₃.

In some specific embodiments of the present invention, the structure of -C(R₁)R₂ or -C(R₃)R₄ in formula (I) independently conforms to the following characteristics, respectively:

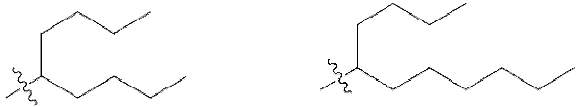

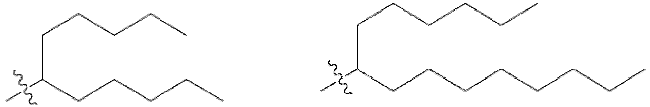

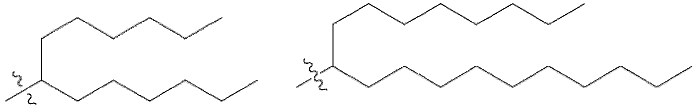

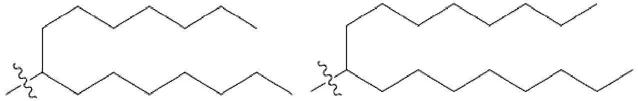

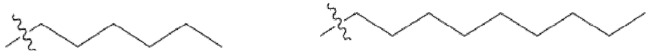

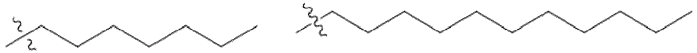

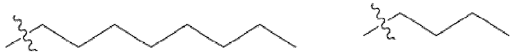

In some specific embodiments of the present invention, the cationic lipid compound has one of the structures shown in the following table:

| No. | Structure |
|---|---|
| 1 | 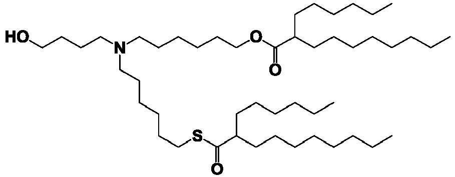 |
| 2 | 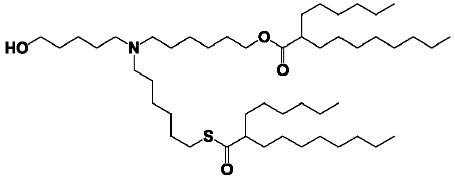 |
| 3 | 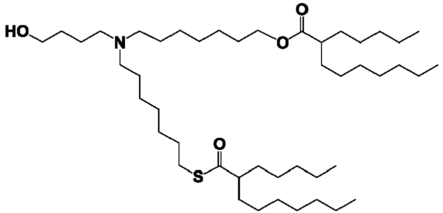 |
| 4 | 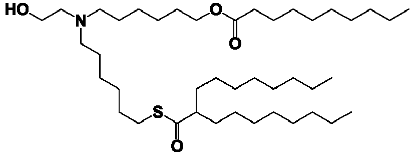 |
| 5 | 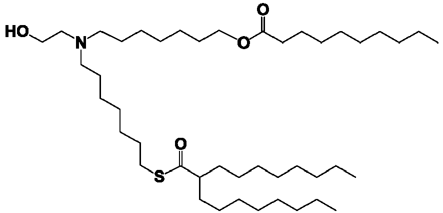 |
| 6 | 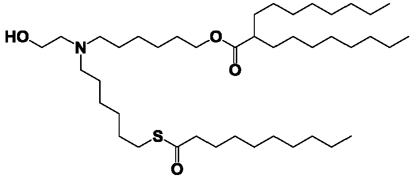 |
| 7 | 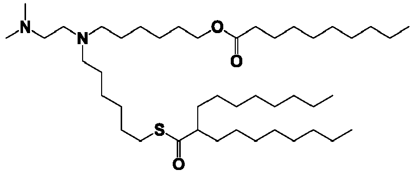 |
| 8 | 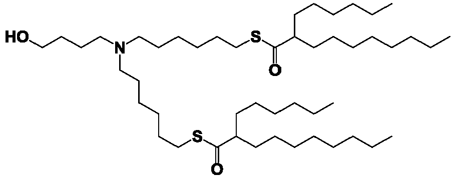 |
| 9 | 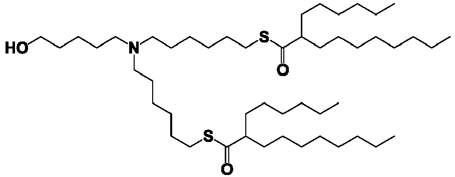 |
| 10 | 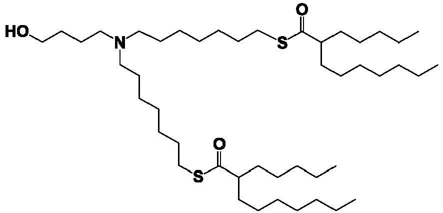 |
| 11 | 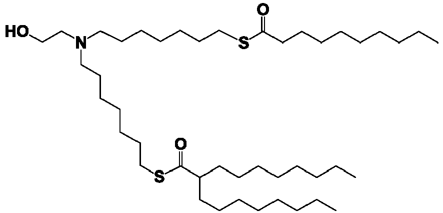 |
| 12 | 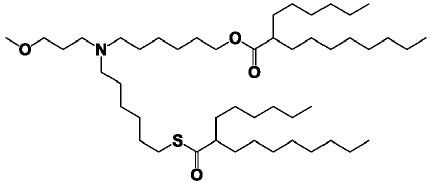 |
| 13 | 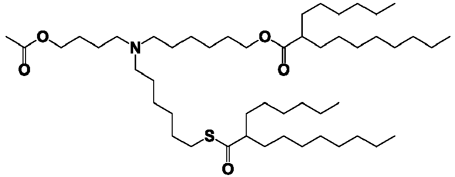 |
| 14 | 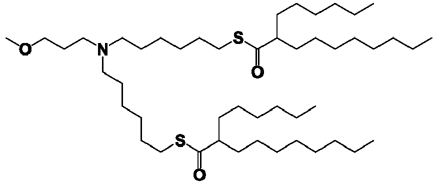 |
| 15 | 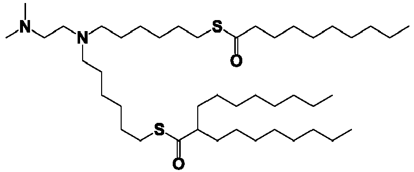 |
| 16 | 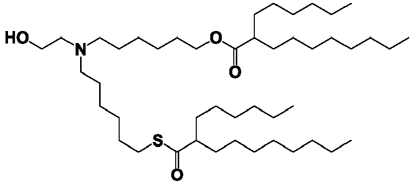 |
| 17 | 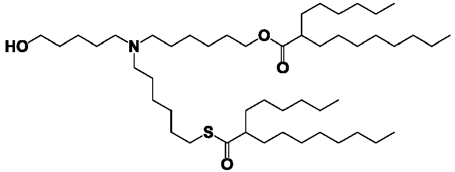 |
| 18 | 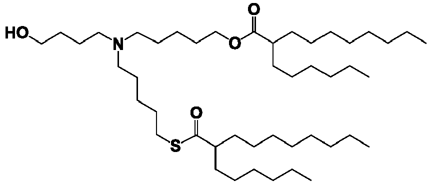 |
| 19 | 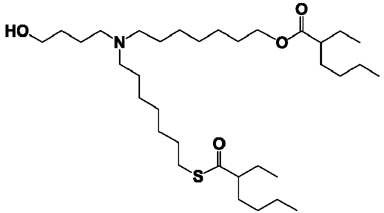 |
| 20 | 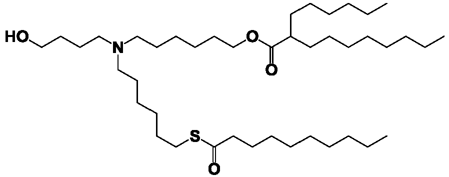 |
| 21 | 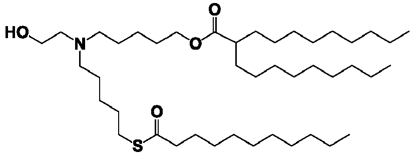 |
| 22 | 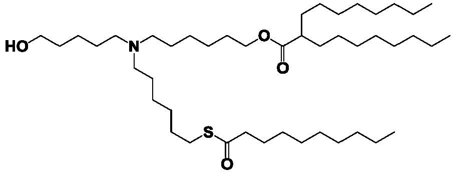 |
| 23 | 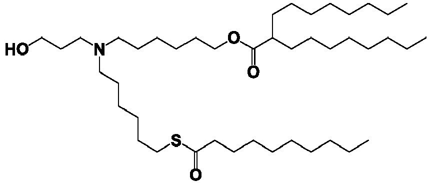 |
| 24 | 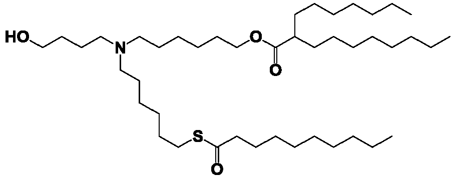 |
| 25 | 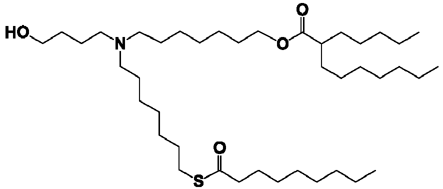 |
| 26 | 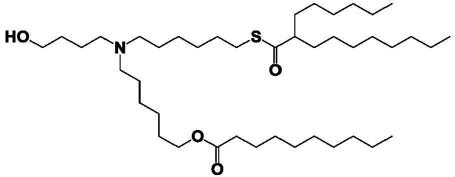 |
| 27 | 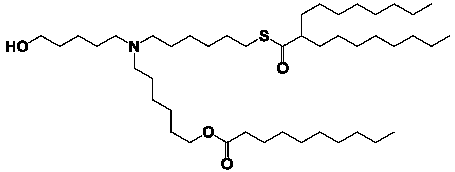 |
| 28 | 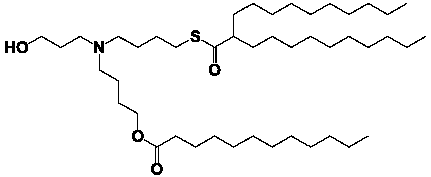 |
| 29 | 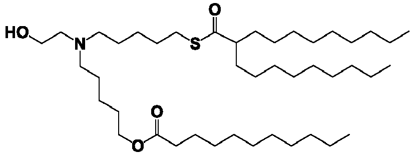 |
| 30 | 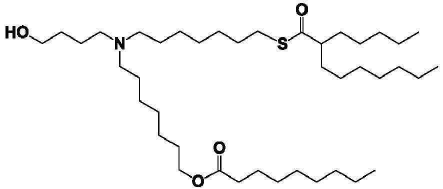 |
| 31 | 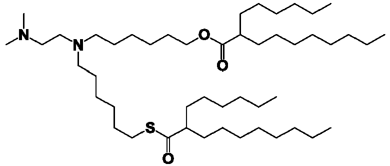 |
| 32 | 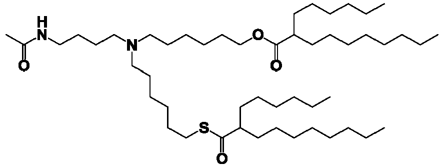 |
| 33 | 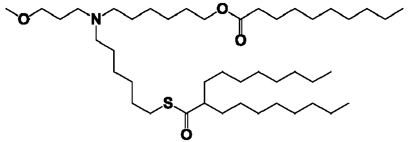 |
| 34 | 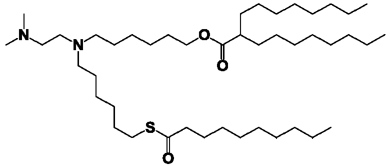 |
| 35 | 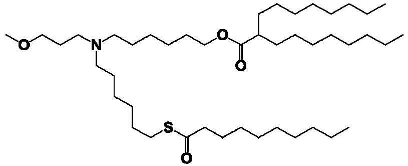 |
| 36 | 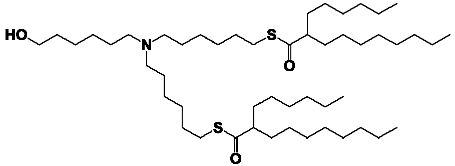 |
| 37 | 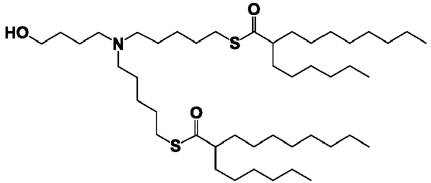 |
| 38 | 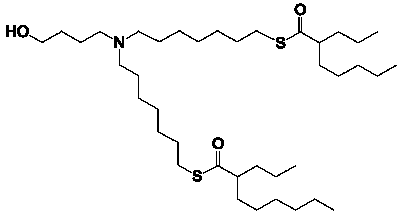 |
| 39 | 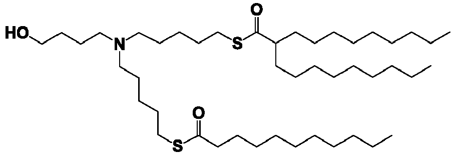 |
| 40 | 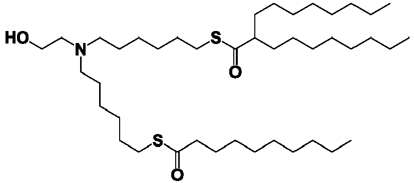 |
| 41 | 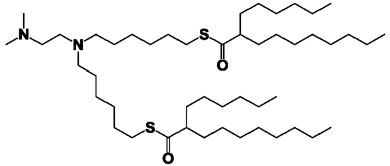 |
| 42 | 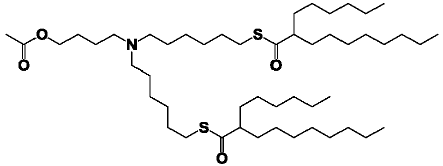 |
| 43 | 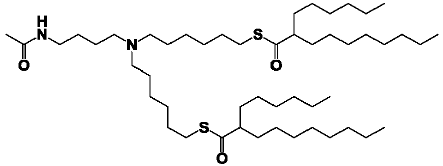 |
| 44 | 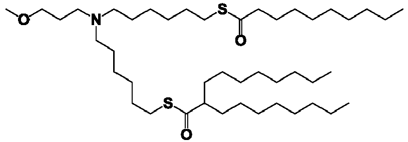 |
| 45 | 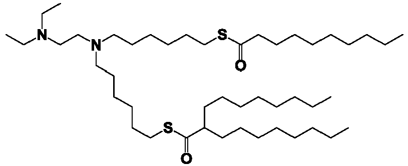 |

The present invention also provides a liposomal formulation comprising one or more cationic lipid compounds of the present invention and prophylactic or therapeutic nucleic acids, wherein the liposomal formulation is for use in the prevention or treatment of certain diseases.

The liposomal formulation comprises one or more components selected from neutral lipids, charged lipids, steroids and polymer-conjugated lipids. The therapeutants used in the present invention are therapeutic nucleic acids comprising plasmid DNA, messenger RNA, antisense oligonucleotide (ASON), micro RNA (miRNA), interfering RNA (micRNA), dicer substrate RNA, complementary DNA (cDNA), preferably plasmid DNA, messenger RNA and antisense oligonucleotides.

In some specific embodiments of the present invention, the molar ratio of the nucleic acid to the cationic lipid compound is from 20:1 to 1:1.

In some specific embodiments of the present invention, the molar ratio of the nucleic acid to the cationic lipid compound is from 10:1 to 4:1.

In some specific embodiments of the present invention, the liposomal formulation has a diameter of 50 nm to 300 nm.

In some specific embodiments of the present invention, the liposomal formulation has a diameter of 50 nm to 150 nm, or 150 nm to 200 nm.

In some specific embodiments of the present invention, it further comprises one or more other lipid components, including, but not limited to, neutral lipids, steroids and polymer-conjugated lipids.

In some specific embodiments of the present invention, the included steroid is cholesterol.

In some specific embodiments of the present invention, the molar ratio of the cholesterol to the cationic lipid compound is (0-1.5): 1.

In some specific embodiments of the present invention, the molar ratio of the cholesterol to the cationic lipid compound is (0.5-1):1, or (1-1.5):1.

In some specific embodiments of the present invention, the polymer in the polymer-conjugated lipid is polyethylene glycol (PEG).

In some specific embodiments of the present invention, the molar ratio of the cationic lipid compound to the polyethylene glycol-conjugated lipid is from 100:1 to 20:1.

In some specific embodiments of the present invention, the molar ratio of the cationic lipid compound to the polyethylene glycol-conjugated lipid is from 50:1 to 25:1.

In some specific embodiments of the present invention, the molar ratio of the cationic lipid compound to the polyethylene glycol-conjugated lipid is from 35:1 to 28:1.

In some specific embodiments of the present invention, the polyethylene glycol-conjugated lipid is PEG-DAG, PEG-PE, PEG-SDAG, PEG-cer, PEG-DMG or ALC-0159.

In some specific embodiments of the present invention, the liposomal formulation comprises one or more neutral lipids selected from DSPC, DSPE, DPPC, DPPG, DMPC, DOPC, POPC, DOPE and SM.

In some specific embodiments of the present invention, the neutral lipid is DSPC or DOPE.

In some specific embodiments of the present invention, the molar ratio of the cationic lipid compound to the neutral lipid is from 2:1 to 8:1.

In some specific embodiments of the present invention, the molar ratio of the cationic lipid compound to the neutral lipid is from 3:1 to 6:1.

In some specific embodiments of the present invention, the molar ratio of the cationic lipid compound to the neutral lipid is from 4:1 to 5:1.

In some specific embodiments of the present invention, the liposomal formulation comprises a nucleic acid.

In some specific embodiments of the present invention, the nucleic acid is selected from antisense RNA and/or messenger RNA.

In some specific embodiments of the present invention, the nucleic acid is messenger RNA.

The present invention also provides the use of the cationic lipid compound or the liposomal formulation described herein in the preparation of a medicament for inducing protein expression in a subject.

In some specific embodiments of the present invention, the subject is a mammal.

In some specific embodiments of the present invention, the subject is a non-human primate.

In some specific embodiments of the present invention, the subject is human.

In summary, the present invention provides a cationic lipid compound and liposomal formulation for delivery of nucleic acids and use thereof. The technical solution of the present invention has the following advantages:
The cationic lipid compound of the present invention has a thioester bond. The introduction of the thioester bond makes the compound more easily degradable and improves the rate of *in vivo* scavenging rate of the lipid compounds, resulting in lower toxicity and fewer residues *in vivo* of the carriers comprising the compounds. Moreover, the method of preparing the aminolipid compounds described herein has the advantages of easy availability of raw materials, mild reaction conditions, high product yield, low requirements for instrumentation and simple operation.

### Brief Description of Drawings

FIG. 1 shows the NMR spectrum of Example 1.
FIG. 2 shows the fluorescence detection results of Example 17.
FIG. 3 shows the measurement results of packed cell volume of Example 18.
FIG. 4 shows the results of the mouse serum neutralizing antibody titer test of Example 19.
FIG. 5 shows a graph containing the content percentage of IFNγ + /TNFα + CD8 + T cells of Example 20.
FIG. 6 shows a graph of changes in tumor volume in mice after tumor transplantation of Example 20.
FIG. 7 shows a graph of the change in body weight of the post-infection mice of Example 21.
FIG. 8 shows a graph of viral load in the right lung of the post-infection mice of Example 21.
FIG. 9 shows the survival rate of the post-infection mice of Example 21.
FIG. 10 shows the results of the neutralizing antibody titer of the post-immunization mice of Example 22.
FIG. 11 shows a graph of cellular immunity level of Example 22.

### Detailed Description of Preferred Embodiments

The technical solutions of the present invention are described in detail below in conjunction with the accompanying drawings and examples, but the protection scope of the present invention includes, but is not limited to, them.

### Example 1 - Synthesis of Compound 1

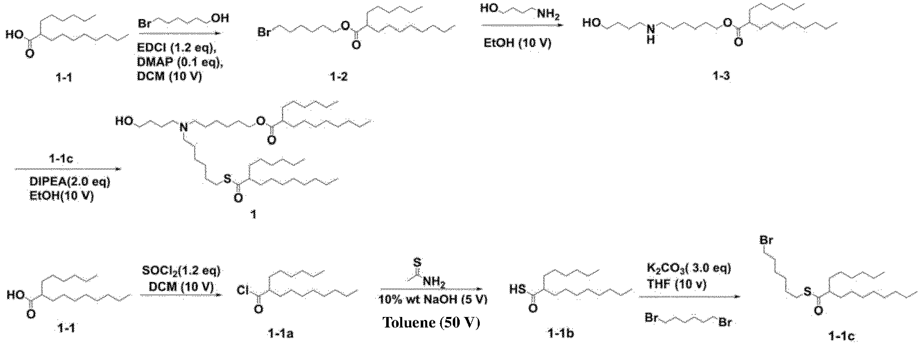

### Step 1:

To a solution of 2-hexyl decanoic acid (1-1, 2.00 g) in DCM (20 mL), 4-dimethylaminopyridine (DMAP, 95 mg) and 6-bromohexanol (1.68 g) were added sequentially. The mixture was stirred at 25°C for 5 mins before 1-ethyl-(3-dimethylaminopropyl) (EDCl, 1.45 g) was added. The reaction mixture was stirred at 25°C for 12 h. Subsequently, TLC showed complete disappearance of the starting alcohol. The reaction mixture was diluted with DCM (30 mL) and washed with saturated NaHCO₃ (100 mL) and brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-2 (2.00 g, 58% yield).

### Step 2:

To a solution of compound 1-2 (2.00 g) in ethanol (20 mL), 4-aminobutan-1-ol (0.47 g) was added. The mixture was stirred at 90°C for 12 h. Subsequently, TLC showed complete disappearance of the starting compound 1-2. The reaction mixture was concentrated to give the crude product, which was purified by column chromatography (with a silica gel column and a n-hexane solution containing 0-1% EA (v/v) as the eluent), and the pure product fraction was evaporated to give a compound 1-3 (1.83 g, 90% yield).

### Step 3:

To a solution of compound 1-1 (4.00 g) in DCM (40 mL), thionyl chloride (2.23 g) was added. The mixture was stirred at 40°C for 6 h. Subsequently, TLC showed complete disappearance of the starting compound 1-1. The reaction mixture was concentrated to give the crude product 1-1a (4.06 g, 95% yield), which was used directly for the next step.

### Step 4:

To a mixed solution of compound 1-1a (4.06 g) in 10 wt% NaOH solution (20 mL) and toluene (200 mL), thioacetamide (1.14 g) was added and stirred at 25°C for 48 h. Subsequently, TLC showed complete disappearance of the starting compound 1-1a. The reaction mixture was extracted twice with EA (30 mL). The organic layers were combined and washed with saturated brine (100 mL). The organic layers combined and dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-lb (2.80 g, 70% yield).

### Step 5:

To a solution of compound 1-1b (2.80 g) in THF (28 mL), potassium carbonate (4.26 g) and 1,6-dibromohexane (2.72 g) were added. The mixture was stirred at 25°C for 12 h. Subsequently, TLC showed complete disappearance of the starting compound 1-1b. The reaction mixture was diluted with water (50 mL) and extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-1c (2.23 g, 50% yield).

### Step 6:

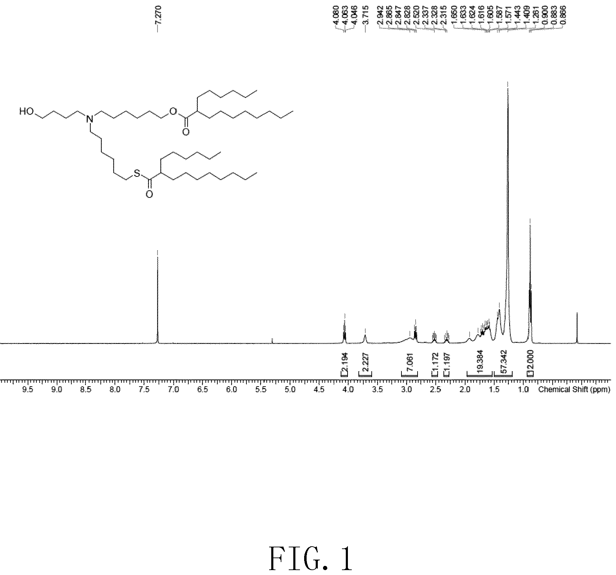

To a solution of compound 1-3 (1.80 g) in ethanol (18 mL), diisopropylethylamine (DIPEA, 1.09 g) and compound 1-1c (2.10 g) were added. The mixture was stirred at 90°C for 12 h. Subsequently, TLC showed complete disappearance of the starting compound 1-3. The reaction mixture was concentrated to give the crude product, which was purified by column chromatography (with a silica gel column and a n-hexane solution containing 0-1% EA (v/v) as the eluent), and the pure product fraction was evaporated to give the compound 1 (2.30 g, 70% yield). The NMR spectrum thereof is shown in Fig. 1.

### Example 2 - Synthesis of Compound 2

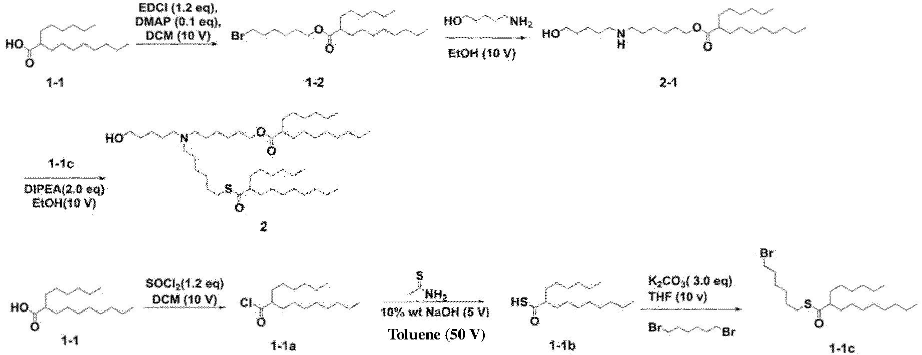

### Step 1:

To a solution of 2-hexyl decanoic acid (1-1, 2.00 g) in DCM (20 mL), 4-dimethylaminopyridine (DMAP, 95 mg) and 6-bromohexanol (1.68 g) were added sequentially. The mixture was stirred at 25°C for 5 mins before 1-ethyl-(3-dimethylaminopropyl) (EDCl, 1.45 g) was added. The reaction mixture was stirred at 25°C for 12 h. Subsequently, TLC showed complete disappearance of the starting alcohol. The reaction mixture was diluted with DCM (30 mL) and washed with saturated NaHCO₃ (100 mL) and brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-2 (2.01 g, 56% yield).

### Step 2:

To a solution of compound 1-2 (2.01 g) in ethanol (20 mL), 4-aminohexan-1-ol (0.54 g) was added. The mixture was stirred at 90°C for 12 h. Subsequently, TLC showed complete disappearance of the starting compound 1-2. The reaction mixture was concentrated to give the crude product, which was purified by column chromatography (with a silica gel column and a n-hexane solution containing 0-1% EA (v/v) as the eluent), and the pure product fraction was evaporated to give a compound 2-3 (1.91 g, 89% yield).

### Step 3:

To a solution of compound 1-1 (4.00 g) in DCM (40 mL), thionyl chloride (2.23 g) was added. The mixture was stirred at 40°C for 6 h. Subsequently, TLC showed complete disappearance of the starting compound 1-1. The reaction mixture was concentrated to give the crude product 1-1a (4.07 g, 96% yield), which was used directly for the next step.

### Step 4:

To a mixed solution of compound 1-1a (4.06 g) in 10 wt% NaOH solution (20 mL) and toluene (200 mL), thioacetamide (1.14 g) was added and stirred at 25°C for 48 h. Subsequently, TLC showed complete disappearance of the starting compound 1-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-lb (2.80 g, 70% yield).

### Step 5:

To a solution of compound 1-1b (2.80 g) in THF (28 mL), potassium carbonate (4.26 g) and 1,6-dibromohexane (2.72 g) were added. The mixture was stirred at 25°C for 12 h. Subsequently, TLC showed complete disappearance of the starting compound 1-1b. The reaction mixture was diluted with water (50 mL) and extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-1c (2.24 g, 51% yield).

### Step 6:

To a solution of compound 2-1 (1.91 g) in ethanol (18 mL), diisopropylethylamine (DIPEA, 1.09 g) and compound 1-1c (2.06 g) were added. The mixture was stirred at 90°C for 12 h. Subsequently, TLC showed complete disappearance of the starting compound 2-1. The reaction mixture was concentrated to give the crude product, which was purified by column chromatography (with a silica gel column and a n-hexane solution containing 0-1% EA (v/v) as the eluent), and the pure product fraction was evaporated to give the compound 2 (2.40 g, 70% yield). ¹H NMR (400 MHz, Chloroform-d) δ 4.07 (t, *J* = 6.5 Hz, 2H), 3.56 (q, *J* = 5.8 Hz, 2H), 3.00 - 2.73 (m, 2H), 2.67 - 2.48 (m, 2H), 2.37 (dt, *J* = 12.3, 6.1 Hz, 6H), 2.29 (p, *J* = 7.7 Hz, 1H), 1.71 - 1.45 (m, 19H), 1.45 - 1.21 (m, 48H), 0.97 - 0.83 (m, 12H).

### Example 3 - Synthesis of Compound 3

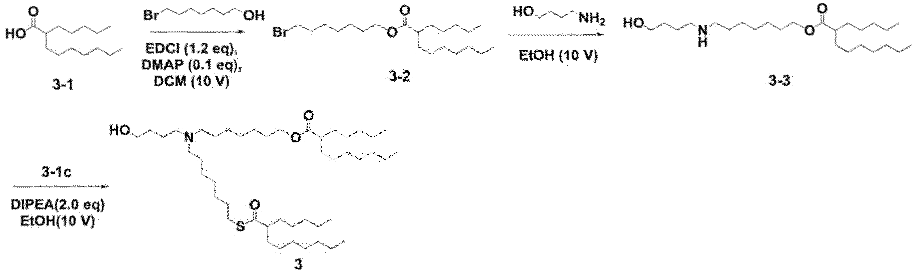

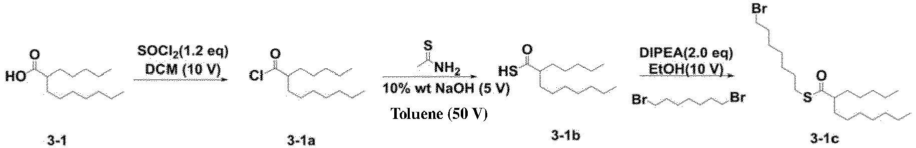

### Step 1:

To a solution of compound 3-1 (2.00 g) in DCM (20 mL), 4-dimethylaminopyridine (DMAP, 84 mg) and 7-bromoheptanol (2.05 g) were added sequentially. The mixture was stirred at 25°C for 5 min before 1-ethyl-(3-dimethylaminopropyl) (EDCl, 1.29 g) was added. The reaction mixture was stirred at 25°C for 12 h. Subsequently, TLC showed complete disappearance of the starting alcohol. The reaction mixture was diluted with DCM (30 mL) and washed with saturated NaHCO₃ (100 mL) and brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 3-2 (2.06 g, 59% yield).

### Step 2:

To a solution of compound 3-2 (2.06 g) in ethanol (20 mL), 4-aminobutan-1-ol (0.50 g) was added. The mixture was stirred at 90°C for 12 h. Subsequently, TLC showed complete disappearance of the starting compound 1-2. The reaction mixture was concentrated to give the crude product, which was purified by column chromatography (with a silica gel column and a n-hexane solution containing 0-1% EA (v/v) as the eluent), and the pure product fraction was evaporated to give a compound 3-3 (1.92 g, 91% yield).

### Step 3:

To a solution of compound 3-1 (4.00 g) in DCM (40 mL), thionyl chloride (2.50 g) was added. The mixture was stirred at 40°C for 6 h. Subsequently, TLC showed complete disappearance of the starting compound 1-1. The reaction mixture was concentrated to give the crude product 3-1a (4.11 g, 94% yield), which was used directly for the next step.

### Step 4:

To a mixed solution of compound 3-1a (4.11 g) in 10 wt% NaOH solution (20 mL) and toluene (200 mL), thioacetamide (1.38 g) was added and stirred at 25°C for 48 h. Subsequently, TLC showed complete disappearance of the starting compound 3-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 3-1b (2.85 g, 71% yield).

### Step 5:

To a solution of compound 3-1b (2.85 g) in THF (28 mL), diisopropylethylamine (DIPEA, 3.01 g) and 1,7-dibromoheptane (3.31 g) were added. The mixture was stirred at 25°C for 12 h. Subsequently, TLC showed complete disappearance of the starting compound 1-1b. The reaction mixture was concentrated and the solvent was removed in vacuum to give the crude product, which was purified by column chromatography (with a silica gel column and a n-hexane solution containing 0-1% EA (v/v) as the eluent), and the pure product fraction was evaporated to give a compound 3-1c (2.46 g, 51% yield).

### Step 6:

To a solution of compound 3-3 (1.92 g) in ethanol (18 mL), diisopropylethylamine (DIPEA, 1.18 g) and compound 3-1c (2.13 g) were added. The mixture was stirred at 90°C for 12 h. Subsequently, TLC showed complete disappearance of the starting compound 2-1. The reaction mixture was concentrated to give the crude product, which was purified by column chromatography (with a silica gel column and a n-hexane solution containing 0-1% EA (v/v) as the eluent), and the pure product fraction was evaporated to give the compound 3 (2.45 g, 72% yield). ¹H NMR (400 MHz, Chloroform-d) δ 4.07 (t, *J* = 6.5 Hz, 2H), 3.61 - 3.54 (m, 2H), 2.95 - 2.80 (m, 2H), 2.58 (p, *J* = 7.0 Hz, 1H), 2.49 - 2.44 (m, 2H), 2.36 (t, *J* = 6.2 Hz, 4H), 2.29 (p, *J* = 7.7 Hz, 1H), 1.66 (ddd, *J* = 14.0, 7.6, 6.4 Hz, 2H), 1.63 - 1.55 (m, 10H), 1.55 - 1.44 (m, 8H), 1.44 - 1.36 (m, 9H), 1.36 - 1.24 (m, 35H), 0.93 - 0.85 (m, 12H).

### Example 4 - Synthesis of Compound 4

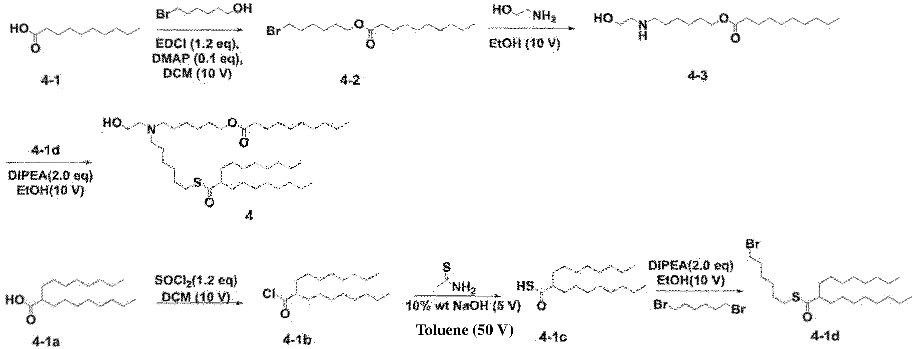

### Step 1:

To a solution of compound 4-1 (2.00 g) in DCM (20 mL), 4-dimethylaminopyridine (DMAP, 141 mg) and 6-bromohexanol (2.52 g) were added sequentially. The mixture was stirred at 25°C for 5 mins before 1-ethyl-(3-dimethylaminopropyl) (EDCl, 2.16 g) was added. The reaction mixture was stirred at 25°C for 12 h. Subsequently, TLC showed complete disappearance of the starting alcohol. The reaction mixture was diluted with DCM (30 mL) and washed with saturated NaHCO₃ (100 mL) and brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 4-2 (2.26 g, 56% yield).

### Step 2:

To a solution of compound 4-2 (2.26 g) in ethanol (20 mL), 2-aminoethan-1-ol (0.45 g) was added. The mixture was stirred at 90°C for 12 h. Subsequently, TLC showed complete disappearance of the starting compound 1-2. The reaction mixture was concentrated to give the crude product, which was purified by column chromatography (with a silica gel column and a n-hexane solution containing 0-1% EA (v/v) as the eluent), and the pure product fraction was evaporated to give a compound 4-3 (1.90 g, 93% yield).

### Step 3:

To a solution of compound 4-1a (4.00 g) in DCM (40 mL), thionyl chloride (2.00 g) was added. The mixture was stirred at 40°C for 6 h. Subsequently, TLC showed complete disappearance of the starting compound 1-1. The reaction mixture was concentrated to give the crude product 4-1b (4.05 g, 93% yield), which was used directly for the next step.

### Step 4:

To a mixed solution of compound 4-1b (4.05 g) in 10 wt% NaOH solution (20 mL) and toluene (200 mL), thioacetamide (1.21 g) was added and stirred at 25°C for 48 h. Subsequently, TLC showed complete disappearance of the starting compound 3-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 4-1c (2.81 g, 72% yield).

### Step 5:

To a solution of compound 4-1c (2.81 g) in THF (28 mL), diisopropylethylamine (DIPEA, 2.41 g) and 1,6-dibromohexane (2.51 g) were added. The mixture was stirred at 25°C for 12 h. Subsequently, TLC showed complete disappearance of the starting compound 1-1b. The reaction mixture was concentrated and the solvent was removed in vacuum to give the crude product, which was purified by column chromatography (with a silica gel column and a n-hexane solution containing 0-1% EA (v/v) as the eluent), and the pure product fraction was evaporated to give a compound 4-1d (2.17 g, 51% yield).

### Step 6:

To a solution of compound 4-3 (1.20 g) in ethanol (18 mL), diisopropylethylamine (DIPEA, 0.98 g) and compound 4-1d (2.13 g) were added. The mixture was stirred at 90°C for 12 h. Subsequently, TLC showed complete disappearance of the starting compound 4-3. The reaction mixture was concentrated to give the crude product, which was purified by column chromatography (with a silica gel column and a n-hexane solution containing 0-1% EA (v/v) as the eluent), and the pure product fraction was evaporated to give the compound **4** (2.44 g, 71% yield). ¹H NMR (400 MHz, Chloroform-*d*) δ 4.03 (t, *J* = 6.2 Hz, 2H), 3.64 (dt, *J* = 6.5, 5.7 Hz, 2H), 2.87 (t, *J* = 6.7 Hz, 2H), 2.82 (t, *J* = 6.3 Hz, 1H), 2.63 - 2.54 (m, 3H), 2.47 (t, *J* = 6.1 Hz, 4H), 2.31 (t, *J* = 8.5 Hz, 2H), 1.68 - 1.45 (m, 14H), 1.45 - 1.22 (m, 42H), 0.97 - 0.81 (m, 9H)_{∘}

### Example 5 - Synthesis of Compound 5

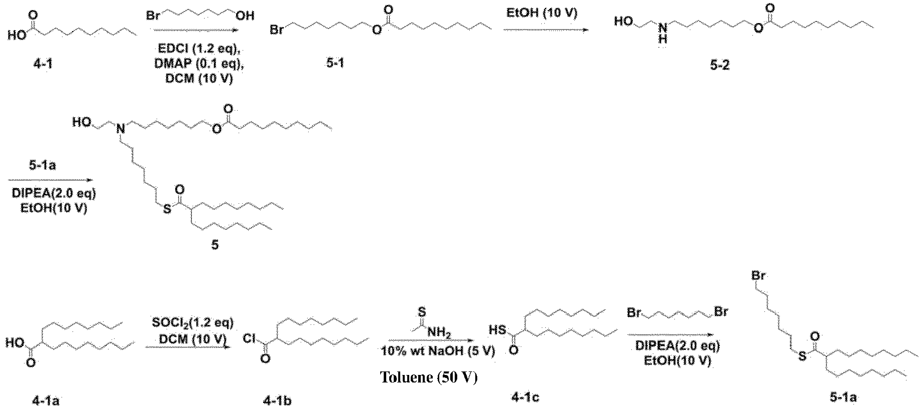

### Step 1:

To a solution of compound 4-1 (2.00 g) in DCM (20 mL), 4-dimethylaminopyridine (DMAP, 142 mg) and 7-bromoheptanol (2.72 g) were added sequentially. The mixture was stirred at 25°C for 5 mins before 1-ethyl-(3-dimethylaminopropyl) (EDCl, 2.16 g) was added. The reaction mixture was stirred at 25°C for 12 h. Subsequently, TLC showed complete disappearance of the starting alcohol. The reaction mixture was diluted with DCM (30 mL) and washed with saturated NaHCO₃ (100 mL) and brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 5-1 (2.35 g, 56% yield).

### Step 2:

To a solution of compound 5-1 (2.35 g) in ethanol (20 mL), 2-aminoethan-1-ol (0.45 g) was added. The mixture was stirred at 90°C for 12 h. Subsequently, TLC showed complete disappearance of the starting compound 1-2. The reaction mixture was concentrated to give the crude product, which was purified by column chromatography (with a silica gel column and a n-hexane solution containing 0-1% EA (v/v) as the eluent), and the pure product fraction was evaporated to give a compound 5-2 (1.99 g, 91% yield).

### Step 3:

To a solution of compound 4-1a (4.00 g) in DCM (40 mL), thionyl chloride (2.00 g) was added. The mixture was stirred at 40°C for 6 h. Subsequently, TLC showed complete disappearance of the starting compound 1-1. The reaction mixture was concentrated to give the crude product 4-1b (4.05 g, 93% yield), which was used directly for the next step.

### Step 4:

To a mixed solution of compound 4-1b (4.05 g) in 10 wt% NaOH solution (20 mL) and toluene (200 mL), thioacetamide (1.21 g) was added and stirred at 25°C for 48 h. Subsequently, TLC showed complete disappearance of the starting compound 3-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 4-1c (2.81 g, 71% yield).

### Step 5:

To a solution of compound 4-1c (2.81 g) in THF (28 mL), diisopropylethylamine (DIPEA, 2.41 g) and 1,7-dibromoheptane (2.65 g) were added. The mixture was stirred at 25°C for 12 h. Subsequently, TLC showed complete disappearance of the starting compound 1-1b. The reaction mixture was concentrated and the solvent was removed in vacuum to give the crude product, which was purified by column chromatography (with a silica gel column and a n-hexane solution containing 0-1% EA (v/v) as the eluent), and the pure product fraction was evaporated to give a compound 5-1c (2.23 g, 51% yield).

### Step 6:

To a solution of compound 5-2 (1.00 g) in ethanol (10 mL), diisopropylethylamine (DIPEA, 0.78 g) and compound 4-1d (1.59 g) were added. The mixture was stirred at 90°C for 12 h. Subsequently, TLC showed complete disappearance of the starting compound 4-3. The reaction mixture was concentrated to give the crude product, which was purified by column chromatography (with a silica gel column and a n-hexane solution containing 0-1% EA (v/v) as the eluent), and the pure product fraction was evaporated to give the compound **3** (2.47 g, 73% yield). ¹H NMR (400 MHz, Chloroform-*d*) δ 4.03 (t, *J* = 6.2 Hz, 2H), 3.64 (dt, *J* = 6.5, 5.7 Hz, 2H), 2.87 (t, *J* = 6.7 Hz, 2H), 2.82 (t, *J* = 6.3 Hz, 1H), 2.62 - 2.54 (m, 3H), 2.47 (t, *J* = 6.1 Hz, 4H), 2.31 (t, *J* = 8.5 Hz, 2H), 1.65 - 1.45 (m, 14H), 1.44 - 1.21 (m, 46H), 0.93 - 0.84 (m, 9H).

### Example 6 - Synthesis of Compound 6

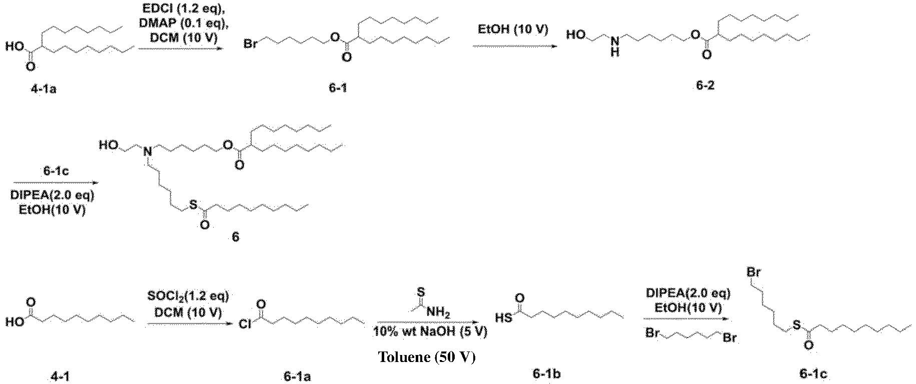

### Step 1:

To a solution of compound 4-1a (2.00 g) in DCM (20 mL), 4-dimethylaminopyridine (DMAP, 86 mg) and 6-bromohexanol (1.53 g) were added sequentially. The mixture was stirred at 25°C for 5 mins before 1-ethyl-(3-dimethylaminopropyl) (EDCl, 1.33 g) was added. The reaction mixture was stirred at 25°C for 12 h. Subsequently, TLC showed complete disappearance of the starting alcohol. The reaction mixture was diluted with DCM (30 mL) and washed with saturated NaHCO₃ (100 mL) and brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 6-1 (1.82 g, 56% yield).

### Step 2:

To a solution of compound 6-1 (1.82 g) in ethanol (20 mL), 4-aminoethan-1-ol (0.27 g) was added. The mixture was stirred at 90°C for 12 h. Subsequently, TLC showed complete disappearance of the starting compound 1-2. The reaction mixture was concentrated to give the crude product, which was purified by column chromatography (with a silica gel column and a n-hexane solution containing 0-1% EA (v/v) as the eluent), and the pure product fraction was evaporated to give a compound 6-2 (1.57 g, 90% yield).

### Step 3:

To a solution of compound 4-1 (4.00 g) in DCM (40 mL), thionyl chloride (3.32 g) was added. The mixture was stirred at 40°C for 6 h. Subsequently, TLC showed complete disappearance of the starting compound 4-1. The reaction mixture was concentrated to give the crude product 6-1a (4.0 g, 95% yield), which was used directly for the next step.

### Step 4:

To a mixed solution of compound 6-1a (4.0g) in 10 wt% NaOH solution (20 mL) and toluene (200 mL), thioacetamide (1.73 g) was added and stirred at 25°C for 48 h. Subsequently, TLC showed complete disappearance of the starting compound 1-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 6-1b (3.0 g, 75% yield).

### Step 5:

To a solution of compound 6-1b (2.80 g) in THF (28 mL), potassium carbonate (6.16 g) and 1,6-dibromohexane (3.81 g) were added. The mixture was stirred at 25°C for 12 h. Subsequently, TLC showed complete disappearance of the starting compound 1-1b. The reaction mixture was diluted with water (50 mL) and extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 6-1c (3.00 g, 59% yield).

### Step 6:

To a solution of compound 6-2 (1.57 g) in ethanol (10 mL), diisopropylethylamine (DIPEA, 0.95 g) and compound 6-1c (1.42 g) were added. The mixture was stirred at 90°C for 12 h. Subsequently, TLC showed complete disappearance of the starting compound 4-3. The reaction mixture was concentrated to give the crude product, which was purified by column chromatography (with a silica gel column and a n-hexane solution containing 0-1% EA (v/v) as the eluent), and the pure product fraction was evaporated to give the compound **6** (1.62 g, 74% yield). ¹H NMR (400 MHz, Chloroform-d) δ 4.07 (t, *J* = 6.5 Hz, 2H), 3.64 (dt, *J* = 6.5, 5.7 Hz, 2H), 2.90 (t, *J* = 6.4 Hz, 2H), 2.82 (t, *J* = 6.3 Hz, 1H), 2.57 (t, *J* = 5.7 Hz, 2H), 2.53 - 2.44 (m, 6H), 2.28 (q, *J* = 7.7 Hz, 1H), 1.71 - 1.45 (m, 14H), 1.45 - 1.24 (m, 43H), 0.93 - 0.85 (m, 9H).

### Example 7 - Synthesis of Compound 7

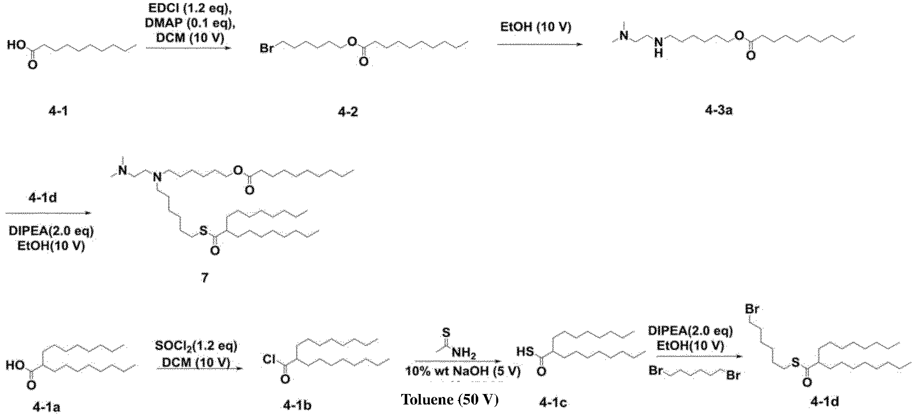

### Step 1:

To a solution of compound 4-1 (2.00 g) in DCM (20 mL), 4-dimethylaminopyridine (DMAP, 141 mg) and 6-bromohexanol (2.52 g) were added sequentially. The mixture was stirred at 25°C for 5 mins before 1-ethyl-(3-dimethylaminopropyl) (EDCl, 2.16 g) was added. The reaction mixture was stirred at 25°C for 12 h. Subsequently, TLC showed complete disappearance of the starting alcohol. The reaction mixture was diluted with DCM (30 mL) and washed with saturated NaHCO₃ (100 mL) and brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 4-2 (2.26 g, 56% yield).

### Step 2:

To a solution of compound 4-2 (2.26 g) in ethanol (20 mL), N,N-dimethylethylenediamine (0.65 g) was added. The mixture was stirred at 90°C for 12 h. Subsequently, TLC showed complete disappearance of the starting compound 1-2. The reaction mixture was concentrated to give the crude product, which was purified by column chromatography (with a silica gel column and a n-hexane solution containing 0-1% EA (v/v) as the eluent), and the pure product fraction was evaporated to give a compound 4-3a (2.08 g, 90% yield).

### Step 3:

To a solution of compound 4-1a (4.00 g) in DCM (40 mL), thionyl chloride (2.00 g) was added. The mixture was stirred at 40°C for 6 h. Subsequently, TLC showed complete disappearance of the starting compound 1-1. The reaction mixture was concentrated to give the crude product 4-1b (4.05 g, 94% yield), which was used directly for the next step.

### Step 4:

To a mixed solution of compound 4-1b (4.05 g) in 10 wt% NaOH solution (20 mL) and toluene (200 mL), thioacetamide (1.21 g) was added and stirred at 25°C for 48 h. Subsequently, TLC showed complete disappearance of the starting compound 3-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 4-1c (2.81 g, 71% yield).

### Step 5:

To a solution of compound 4-1c (2.81 g) in THF (28 mL), diisopropylethylamine (DIPEA, 2.41 g) and 1,6-dibromohexane (2.51 g) were added. The mixture was stirred at 25°C for 12 h. Subsequently, TLC showed complete disappearance of the starting compound 1-1b. The reaction mixture was concentrated and the solvent was removed in vacuum to give the crude product, which was purified by column chromatography (with a silica gel column and a n-hexane solution containing 0-1% EA (v/v) as the eluent), and the pure product fraction was evaporated to give a compound 4-1d (2.17 g, 52% yield).

### Step 6:

To a solution of compound 4-3a (1.40 g) in ethanol (10 mL), diisopropylethylamine (DIPEA, 1.05 g) and compound 4-1d (1.42 g) were added. The mixture was stirred at 90°C for 12 h. Subsequently, TLC showed complete disappearance of the starting compound 4-3a. The reaction mixture was concentrated to give the crude product, which was purified by column chromatography (with a silica gel column and a n-hexane solution containing 0-1% EA (v/v) as the eluent), and the pure product fraction was evaporated to give the compound 7 (2.07 g, 71% yield). ¹H NMR (500 MHz, Chloroform-*d*) δ 4.03 (t, *J* = 6.2 Hz, 2H), 2.87 (t, *J* = 6.7 Hz, 2H), 2.63 - 2.53 (m, 5H), 2.45 (t, *J* = 6.1 Hz, 4H), 2.35 (s, 6H), 2.31 (t, *J* = 8.5 Hz, 2H), 1.71 - 1.46 (m, 14H), 1.44 - 1.19 (m, 42H), 1.02 - 0.77 (m, 9H)_{∘}

### Example 8 - Synthesis of Compound 8

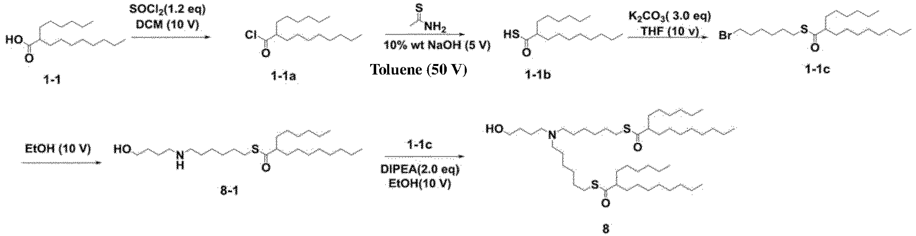

### Step 1:

To a solution of compound 1-1 (2.00 g) in DCM (20 mL), thionyl chloride (1.16 g) was added. The mixture was stirred at 40°C for 6 h. Subsequently, TLC showed complete disappearance of the starting compound 1-1. The reaction mixture was concentrated to give the crude product 1-1a (2.03 g, 96% yield), which was used directly for the next step.

### Step 2:

To a mixed solution of compound 1-1a (2.03 g) in 10 wt% NaOH solution (10 mL) and toluene (100 mL), thioacetamide (0.57 g) was added and stirred at 25°C for 48 h. Subsequently, TLC showed complete disappearance of the starting compound 1-1a. The reaction mixture was extracted twice with EA (30 mL). The organic layers were combined and washed with saturated brine (200 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-lb (1.40 g, 72% yield).

### Step 3:

To a solution of compound 1-1b (1.40 g) in THF (28 mL), potassium carbonate (2.13 g) and 1,6-dibromohexane (1.36 g) were added. The mixture was stirred at 25°C for 12 h. Subsequently, TLC showed complete disappearance of the starting compound 1-1b. The reaction mixture was diluted with water (25 mL) and extracted twice with EA (15 mL). The organic layers were combined and washed with saturated brine (50 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-1c (1.17 g, 51% yield).

### Step 4:

To a solution of compound 1-1c (1.17 g) in ethanol (10 mL), diisopropylethylamine (DIPEA, 0.69 g) and compound 4-1d (1.42 g) were added. The mixture was stirred at 90°C for 12 h. Subsequently, TLC showed complete disappearance of the starting compound 1-1c. The reaction mixture was concentrated to give the crude product, which was purified by column chromatography (with a silica gel column and a n-hexane solution containing 0-1% EA (v/v) as the eluent), and the pure product fraction was evaporated to give the compound **8** (1.07 g, 49% yield). ¹H NMR (400 MHz, Chloroform-d) δ 3.61 - 3.54 (m, 2H), 2.95 - 2.80 (m, 4H), 2.73 (t, *J* = 5.8 Hz, 1H), 2.58 (p, *J* = 7.0 Hz, 2H), 2.49 - 2.44 (m, 2H), 2.36 (t, *J* = 6.1 Hz, 4H), 1.61 (d, *J* = 6.6 Hz, 3H), 1.59 - 1.53 (m, 13H), 1.52 - 1.45 (m, 4H), 1.45 - 1.40 (m, 4H), 1.40 - 1.30 (m, 20H), 1.30 - 1.23 (m, 23H), 0.94 - 0.85 (m, 12H).

### Example 9 - Synthesis of Compound 9

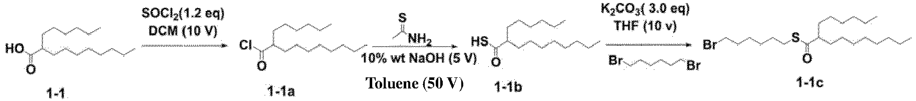

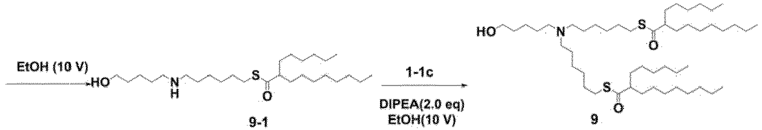

### Step 1:

To a solution of compound 1-1 (4.00 g) in DCM (40 mL), thionyl chloride (2.23 g) was added. The mixture was stirred at 40°C for 6 h. Subsequently, TLC showed complete disappearance of the starting compound 1-1. The reaction mixture was concentrated to give the crude product 1-1a (4.06 g, 95% yield), which was used directly for the next step.

### Step 2:

To a mixed solution of compound 1-1a (4.06 g) in 10 wt% NaOH solution (20 mL) and toluene (200 mL), thioacetamide (1.14 g) was added and stirred at 25°C for 48 h. Subsequently, TLC showed complete disappearance of the starting compound 1-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-lb (2.80 g, 70% yield).

### Step 3:

To a solution of compound 1-1b (2.80 g) in THF (28 mL), potassium carbonate (4.26 g) and 1,6-dibromohexane (2.72 g) were added. The mixture was stirred at 25°C for 12 h. Subsequently, TLC showed complete disappearance of the starting compound 1-1b. The reaction mixture was diluted with water (50 mL) and extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-1c (2.23 g, 50% yield).

### Step 4:

To a solution of compound 1-1c (1.0 g) in ethanol (20 mL), 5-hydroxypentylamine (0.26 g) was added. The mixture was stirred at 90°C for 12 h. Subsequently, TLC showed complete disappearance of the starting compound 1-2. The reaction mixture was concentrated to give the crude product, which was purified by column chromatography (with a silica gel column and a n-hexane solution containing 0-1% EA (v/v) as the eluent), and the pure product fraction was evaporated to give the compound 9-1 (0.8 g, 76% yield).

### Step 5:

To a solution of compound 9-1 (0.8 g) in ethanol (10 mL), diisopropylethylamine (DIPEA, 0.45 g) and compound 1-1c (0.84 g) were added. The mixture was stirred at 90°C for 12 h. Subsequently, TLC showed complete disappearance of the starting compound 9-1. The reaction mixture was concentrated to give the crude product, which was purified by column chromatography (with a silica gel column and a n-hexane solution containing 0-1% EA (v/v) as the eluent), and the pure product fraction was evaporated to give the compound **9** (1.0 g, 70% yield). ¹H NMR (400 MHz, Chloroform-d) δ 3.56 (q, *J* = 5.8 Hz, 1H), 2.95 - 2.80 (m, 2H), 2.63 - 2.54 (m, 2H), 2.37 (dt, *J* = 12.4, 6.1 Hz, 3H), 1.63 - 1.46 (m, 10H), 1.46 - 1.31 (m, 12H), 1.31-1.23 (m, 13H), 0.92 - 0.86 (m, 6H).

### Example 10 - Synthesis of Compound 10

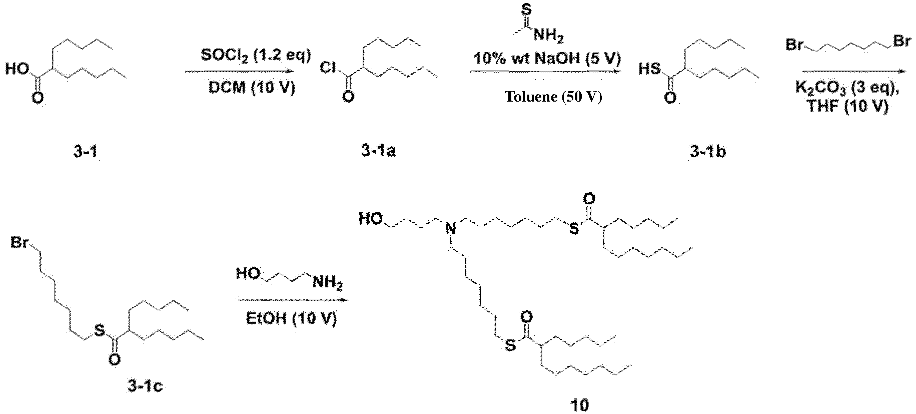

### Step 1:

To a solution of compound 3-1 (4.00 g) in DCM (40 mL), thionyl chloride (2.50 g) was added. The mixture was stirred at 40°C for 6 h. Subsequently, TLC showed complete disappearance of the starting compound 1-1. The reaction mixture was concentrated to give the crude product 3-1a (4.11 g, 95% yield), which was used directly for the next step.

### Step 2:

To a mixed solution of compound 3-1a (4.11 g) in 10 wt% NaOH solution (20 mL) and toluene (200 mL), thioacetamide (1.38 g) was added and stirred at 25°C for 48 h. Subsequently, TLC showed complete disappearance of the starting compound 3-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 3-1b (2.85 g, 70% yield).

### Step 3:

To a solution of compound 3-1b (2.85 g) in THF (28 mL), diisopropylethylamine (DIPEA, 3.01 g) and 1,7-dibromoheptane (3.31 g) were added. The mixture was stirred at 25°C for 12 h. Subsequently, TLC showed complete disappearance of the starting compound 1-1b. The reaction mixture was concentrated and the solvent was removed in vacuum to give the crude product, which was purified by column chromatography (with a silica gel column and a n-hexane solution containing 0-1% EA (v/v) as the eluent), and the pure product fraction was evaporated to give a compound 3-1c (2.46 g, 50% yield).

### Step 4:

To a solution of compound 3-1c (2.00 g) in ethanol (20 mL), 4-hydroxybutylamine (0.26 g) was added. The mixture was stirred at 90°C for 12 h. Subsequently, TLC showed complete disappearance of the starting compound 3-1c. The reaction mixture was concentrated to give the crude product, which was purified by column chromatography (with a silica gel column and a n-hexane solution containing 0-1% EA (v/v) as the eluent), and the pure product fraction was evaporated to give the compound **10** (2.6 g, 66% yield). ¹H NMR (500 MHz, Chloroform-d) δ 3.62 - 3.50 (m, 2H), 2.96 - 2.80 (m, 4H), 2.73 (t, *J* = 5.8 Hz, 1H), 2.58 (p, *J* = 7.0 Hz, 2H), 2.50 - 2.44 (m, 2H), 2.36 (t, *J* = 6.1 Hz, 4H), 1.64 - 1.45 (m, 20H), 1.44 - 1.23 (m, 44H), 0.96 - 0.82 (m, 12H).

### Example 11 - Synthesis of Compound 11

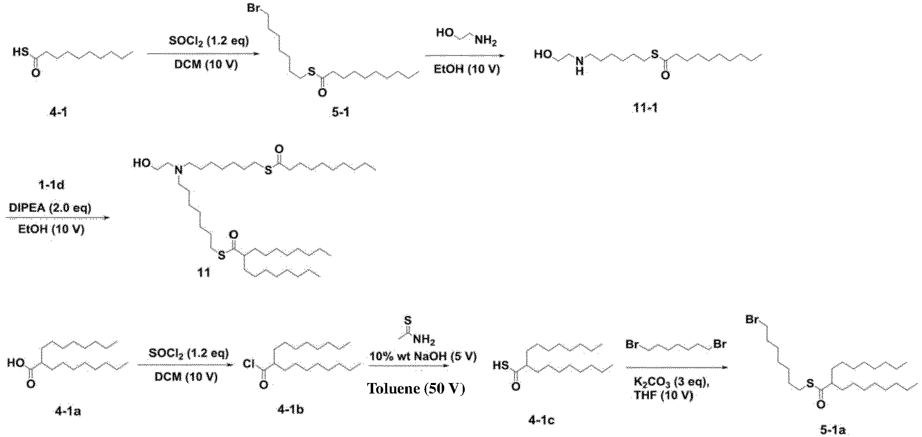

### Step 1:

To a solution of compound 4-1 (2.00 g) in DCM (20 mL), 4-dimethylaminopyridine (DMAP, 142 mg) and 7-bromoheptanol (2.72 g) were added sequentially. The mixture was stirred at 25°C for 5 mins before 1-ethyl-(3-dimethylaminopropyl) (EDCl, 2.16 g) was added. The reaction mixture was stirred at 25°C for 12 h. Subsequently, TLC showed complete disappearance of the starting alcohol. The reaction mixture was diluted with DCM (30 mL) and washed with saturated NaHCO₃ (100 mL) and brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 5-1 (2.35 g, 58% yield).

### Step 2:

To a solution of compound 5-1 (2.00g) in ethanol (20 mL), 2-hydroxyethylamine (0.37 g) was added. The mixture was stirred at 90°C for 12 h. Subsequently, TLC showed complete disappearance of the starting compound 5-1. The reaction mixture was concentrated to give the crude product, which was purified by column chromatography (with a silica gel column and a n-hexane solution containing 0-1% EA (v/v) as the eluent), and the pure product fraction was evaporated to give a compound 11-1(1.30 g, 71% yield).

### Step 3:

To a solution of compound 4-1a (4.00 g) in DCM (40 mL), thionyl chloride (2.00 g) was added. The mixture was stirred at 40°C for 6 h. Subsequently, TLC showed complete disappearance of the starting compound 1-1. The reaction mixture was concentrated to give the crude product 4-1b (4.05 g, 95% yield), which was used directly for the next step.

### Step 4:

To a mixed solution of compound 4-1b (4.05 g) in 10 wt% NaOH solution (20 mL) and toluene (200 mL), thioacetamide (1.21 g) was added and stirred at 25°C for 48 h. Subsequently, TLC showed complete disappearance of the starting compound 3-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 4-1c (2.81 g, 70% yield).

### Step 5:

To a solution of compound 4-1c (2.81 g) in THF (28 mL), diisopropylethylamine (DIPEA, 2.41 g) and 1,7-dibromoheptane (2.65 g) were added. The mixture was stirred at 25°C for 12 h. Subsequently, TLC showed complete disappearance of the starting compound 1-1b. The reaction mixture was concentrated and the solvent was removed in vacuum to give the crude product, which was purified by column chromatography (with a silica gel column and a n-hexane solution containing 0-1% EA (v/v) as the eluent), and the pure product fraction was evaporated to give a compound 5-1a (2.23 g, 50% yield).

### Step 6:

To a solution of compound 11-1 (1.0 g) in ethanol (15 mL), diisopropylethylamine (DIPEA, 0.78 g) and compound 5-1a (1.58 g) were added. The mixture was stirred at 90°C for 12 h. Subsequently, TLC showed complete disappearance of the starting compound 11-1. The reaction mixture was concentrated to give the crude product, which was purified by column chromatography (with a silica gel column and a n-hexane solution containing 0-1% EA (v/v) as the eluent), and the pure product fraction was evaporated to give the compound **11** (1.50 g, 68% yield). ¹H NMR (400 MHz, Chloroform-*d*) δ 3.61 - 3.54 (m, 2H), 2.95 - 2.80 (m, 4H), 2.73 (t, *J* = 5.8 Hz, 1H), 2.58 (m, 2H), 2.50 - 2.44 (m, 2H), 2.36 (t, *J* = 6.1 Hz, 4H), 1.64 - 1.43 (m, 20H), 1.45 - 1.23 (m, 45H), 0.94 - 0.85 (m, 12H).

### Example 12 - Synthesis of Compound 12

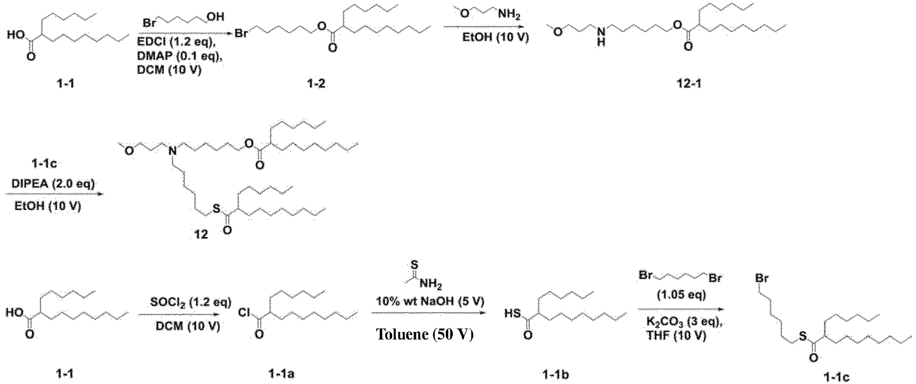

### Step 1:

To a solution of 2-hexyl decanoic acid (1-1, 2.00 g) in DCM (20 mL), 4-dimethylaminopyridine (DMAP, 95 mg) and 6-bromohexanol (1.68 g) were added sequentially. The mixture was stirred at 25°C for 5 mins before 1-ethyl-(3-dimethylaminopropyl) (EDCl, 1.45 g) was added. The reaction mixture was stirred at 25°C for 12 h. Subsequently, TLC showed complete disappearance of the starting alcohol. The reaction mixture was diluted with DCM (30 mL) and washed with saturated NaHCO₃ (100 mL) and brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-2 (2.00 g, 58% yield).

### Step 2:

To a solution of compound 1-2 (1.50 g) in ethanol (20 mL), 3-methoxypropylamine (0.35 g) was added. The mixture was stirred at 90°C for 12 h. Subsequently, TLC showed complete disappearance of the starting compound 1-2. The reaction mixture was concentrated to give the crude product, which was purified by column chromatography (with a silica gel column and a n-hexane solution containing 0-1% EA (v/v) as the eluent), and the pure product fraction was evaporated to give a compound 12-1 (1.20 g, 78% yield).

### Step 3:

To a solution of compound 1-1 (4.00 g) in DCM (40 mL), thionyl chloride (2.23 g) was added. The mixture was stirred at 40°C for 6 h. Subsequently, TLC showed complete disappearance of the starting compound 1-1. The reaction mixture was concentrated to give the crude product 1-1a (4.06 g, 95% yield), which was used directly for the next step.

### Step 4:

To a mixed solution of compound 1-1a (4.06 g) in 10 wt% NaOH solution (20 mL) and toluene (200 mL), thioacetamide (1.41 g) was added and stirred at 25°C for 48 h. Subsequently, TLC showed complete disappearance of the starting compound 1-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-lb (2.80 g, 70% yield).

### Step 5:

To a solution of compound 1-1b (2.80 g) in THF (28 mL), potassium carbonate (4.26 g) and 1,6-dibromohexane (2.72 g) were added. The mixture was stirred at 25°C for 12 h. Subsequently, TLC showed complete disappearance of the starting compound 1-1b. The reaction mixture was diluted with water (50 mL) and extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-1c (2.23 g, 50% yield).

### Step 6:

To a solution of compound 12-1 (1.0 g) in ethanol (15 mL), diisopropylethylamine (DIPEA, 0.87 g) and compound 1-1c (1.20 g) were added. The mixture was stirred at 90°C for 12 h. Subsequently, TLC showed complete disappearance of the starting compound 12-1. The reaction mixture was concentrated to give the crude product, which was purified by column chromatography (with a silica gel column and a n-hexane solution containing 0-1% EA (v/v) as the eluent), and the pure product fraction was evaporated to give the compound **12** (1.2 g, 65% yield). ¹H NMR (400 MHz, Chloroform-*d*) δ 4.07 (t, *J* = 6.5 Hz, 2H), 3.42 (td, *J* = 6.6, 1.4 Hz, 2H), 3.18 (s, 3H), 2.95 - 2.80 (m, 2H), 2.63 - 2.52 (m, 3H), 2.37 (t, *J* = 6.1 Hz, 4H), 2.29 (m, 1H), 1.79 - 1.22 (m, 66H), 0.94 - 0.85 (m, 12H).

### Example 13 - Synthesis of Compound 13

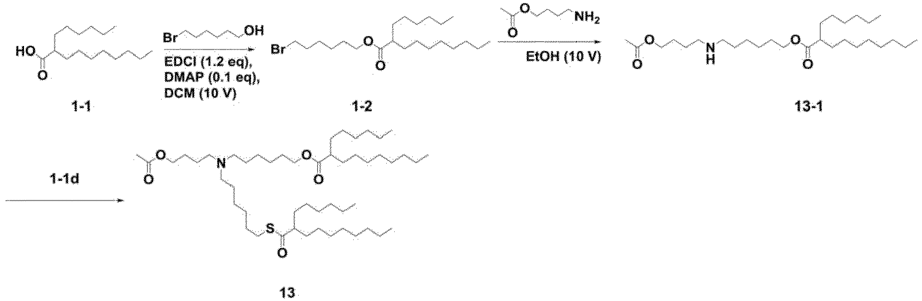

### Step 1:

To a solution of 2-hexyl decanoic acid (1-1, 2.00 g) in DCM (20 mL), 4-dimethylaminopyridine (DMAP, 95 mg) and 6-bromohexanol (1.68 g) were added sequentially. The mixture was stirred at 25°C for 5 mins before 1-ethyl-(3-dimethylaminopropyl) (EDCl, 1.45 g) was added. The reaction mixture was stirred at 25°C for 12 h. Subsequently, TLC showed complete disappearance of the starting alcohol. The reaction mixture was diluted with DCM (30 mL) and washed with saturated NaHCO₃ (100 mL) and brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-2 (2.00 g, 58% yield).

### Step 2:

To a solution of compound 1-2 (2.00 g) in ethanol (20 mL), 4-acetoxybutylamine (0.69 g) was added. The mixture was stirred at 90°C for 12 h. Subsequently, TLC showed complete disappearance of the starting compound 1-2. The reaction mixture was concentrated to give the crude product, which was purified by column chromatography (with a silica gel column and a n-hexane solution containing 0-1% EA (v/v) as the eluent), and the pure product fraction was evaporated to give a compound 13-1 (1.80 g, 80% yield).

### Step 3:

To a solution of compound 13-1 (1.50 g) in ethanol (15 mL), diisopropylethylamine (DIPEA, 0.87 g) and compound 1-1d (1.60 g) were added. The mixture was stirred at 90°C for 12 h. Subsequently, TLC showed complete disappearance of the starting compound 1-3. The reaction mixture was concentrated to give the crude product, which was purified by column chromatography (with a silica gel column and a n-hexane solution containing 0-1% EA (v/v) as the eluent), and the pure product fraction was evaporated to give the compound **13** (2.0 g, 76% yield). ¹H NMR (400 MHz, Chloroform-d) δ 4.11 - 4.04 (dt, *J* = 8.0, 6.4 Hz, 4H), 2.38 - 2.34 (t, *J* = 6.1 Hz, 4H), 2.32 - 2.26 (m, 1H), 2.03 (s, 2H), 1.79 - 1.21 (m, 68H), 0.92 - 0.87 (m, 12H).

### Example 14 - Synthesis of Compound 14

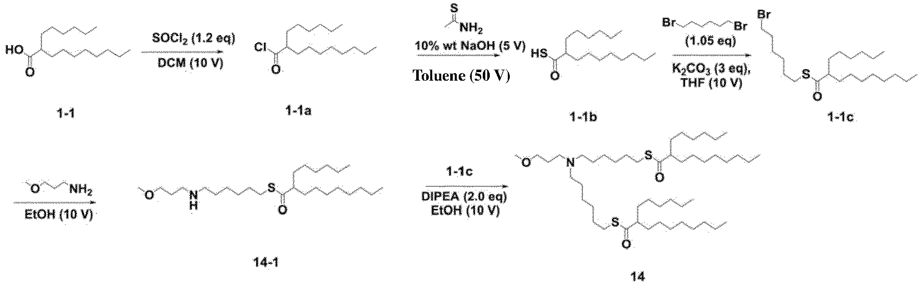

### Step 1:

To a solution of compound 1-1 (4.00 g) in DCM (40 mL), thionyl chloride (2.23 g) was added. The mixture was stirred at 40°C for 6 h. Subsequently, TLC showed complete disappearance of the starting compound 1-1. The reaction mixture was concentrated to give the crude product 1-1a (4.06 g, 95% yield), which was used directly for the next step.

### Step 2:

To a mixed solution of compound 1-1a (4.06 g) in 10 wt% NaOH solution (20 mL) and toluene (200 mL), thioacetamide (1.14 g) was added and stirred at 25°C for 48 h. Subsequently, TLC showed complete disappearance of the starting compound 1-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-lb (2.80 g, 70% yield).

### Step 3:

To a solution of compound 1-1b (2.80 g) in THF (28 mL), potassium carbonate (4.26 g) and 1,6-dibromohexane (2.72 g) were added. The mixture was stirred at 25°C for 12 h. Subsequently, TLC showed complete disappearance of the starting compound 1-1b. The reaction mixture was diluted with water (50 mL) and extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 1-1c (2.23 g, 50% yield).

### Step 4:

To a solution of compound 1-1c (1.0 g) in ethanol (20 mL), 3-methoxypropylamine (0.45 g) was added. The mixture was stirred at 90°C for 12 h. Subsequently, TLC showed complete disappearance of the starting compound 1-1c. The reaction mixture was concentrated to give the crude product, which was purified by column chromatography (with a silica gel column and a n-hexane solution containing 0-1% EA (v/v) as the eluent), and the pure product fraction was evaporated to give the compound 14-1 (1.80 g, 88% yield).

### Step 5:

To a solution of compound 14-1 (1.50 g) in ethanol (18 mL), diisopropylethylamine (DIPEA, 0.87 g) and compound 1-1c (1.62 g) were added. The mixture was stirred at 90°C for 12 h. Subsequently, TLC showed complete disappearance of the starting compound 1-3. The reaction mixture was concentrated to give the crude product, which was purified by column chromatography (with a silica gel column and a n-hexane solution containing 0-1% EA (v/v) as the eluent), and the pure product fraction was evaporated to give the compound **14** (2.10 g, 78% yield). ¹H NMR (400 MHz, Chloroform-d) δ 3.42 (td, *J* = 6.6, 1.4 Hz, 2H), 3.18 (s, 3H), 2.95 - 2.81 (m, 4H), 2.63 - 2.52 (m, 4H), 2.37 (t, *J* = 6.1 Hz, 4H), 1.78 - 1.64 (m, *J* = 6.5 Hz, 2H), 1.63 - 1.23 (m, 64H), 0.92 - 0.85 (m, 12H).

### Example 15 - Synthesis of Compound 15

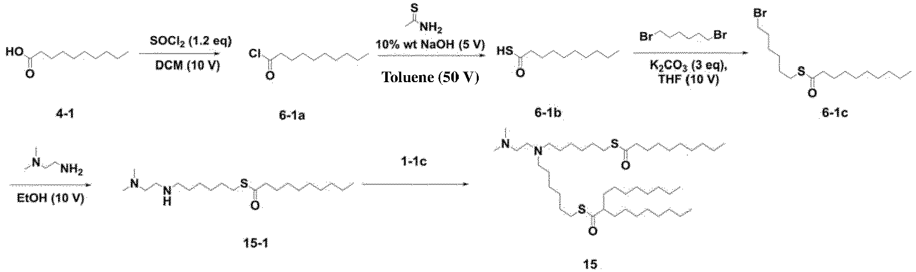

### Step 1:

To a solution of compound 4-1 (4.00 g) in DCM (40 mL), thionyl chloride (3.32 g) was added. The mixture was stirred at 40°C for 6 h. Subsequently, TLC showed complete disappearance of the starting compound 4-1. The reaction mixture was concentrated to give the crude product 6-1a (4.0 g, 90% yield), which was used directly for the next step.

### Step 2:

To a mixed solution of compound 6-1a (4.0 g) in 10 wt% NaOH solution (20 mL) and toluene (200 mL), thioacetamide (1.73 g) was added and stirred at 25°C for 48 h. Subsequently, TLC showed complete disappearance of the starting compound 1-1a. The reaction mixture was extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 6-1b (3.0 g, 76% yield).

### Step 3:

To a solution of compound 6-1b (2.80 g) in THF (28 mL), potassium carbonate (6.16 g) and 1,6-dibromohexane (3.81 g) were added. The mixture was stirred at 25°C for 12 h. Subsequently, TLC showed complete disappearance of the starting compound 1-1b. The reaction mixture was diluted with water (50 mL) and extracted twice with EA (30 mL). The organic layers were combined and washed with saturated brine (100 mL). The combined organic layers were dried over Na₂SO₄, and the solvent was removed in vacuum to give the crude product, which was purified by column chromatography (with a silica gel column and a n-hexane solution containing 0-1% EA (v/v) as the eluent), and the pure product fraction was evaporated to give a compound 6-1c (3.00 g, 57% yield).

### Step 4:

To a solution of compound 6-1c (2.50 g) in ethanol (20 mL), N,N-dimethyl-1,2-ethylenediamine (0.47 g) was added. The mixture was stirred at 90°C for 12 h. Subsequently, TLC showed complete disappearance of the starting compound 1-2. The reaction mixture was concentrated to give the crude product, which was purified by column chromatography (with a silica gel column and a n-hexane solution containing 0-1% EA (v/v) as the eluent), and the pure product fraction was evaporated to give the compound 15-1 (2.30 g, 90% yield).

### Step 5:

To a solution of compound 15-1 (2.00 g) in ethanol (20 mL), N,N-dimethyl-1,2-ethylenediamine (1.44 g) and compound 1-1c (2.84 g) were added. The mixture was stirred at 90°C for 12 h. Subsequently, TLC showed complete disappearance of the starting compound 15-1. The reaction mixture was concentrated to give the crude product, which was purified by column chromatography (with a silica gel column and a n-hexane solution containing 0-1% EA (v/v) as the eluent), and the pure product fraction was evaporated to give the compound 15 (2.6 g, 63% yield). ¹H NMR (400 MHz, Chloroform-*d*) δ 2.92 - 2.85 (dt, *J* = 12.1, 6.5 Hz, 4H), 2.61 - 2.56 (m, 5H), 2.53 - 2.48 (t, *J* = 7.6 Hz, 2H), 2.47 - 2.43 (t, *J* = 6.1 Hz, 4H), 2.38 - 2.34 (s, 6H), 1.64 - 1.57 (m, 7H), 1.57 - 1.51 (m, 4H), 1.50 - 1.45 (m, 4H), 1.44 - 1.25 (m, 43H), 0.92 - 0.87 (m, 9H).

### Example 16 - Preparation of nano-lipid particles and characterization of properties thereof

Preparation of aqueous phase: mRNA was diluted in a citrate buffer for a final concentration of 0.13 µg/µL.

The lipid fractions (cationic lipids described herein, DSPC, cholesterol, PEG lipids) were dissolved in ethanol at a total concentration of 10 mg/mL, and the ratio varied with cationic lipids. The PEG lipid used for Nos. 1-3, 8-10, 12-14 and 16 was ALC-0159; the PEG lipid used for Nos. 4-7, 11, 15 and 17-19 was PEG2000-DMG.

3 mL of a mRNA buffer and 1 mL of a lipid solution were loaded into two 5 mL syringes installed on a microfluidic syringe pump, respectively,. Chips were connected to the syringes and syringe pump flow rate was set. Clicking the start button of the syringe pump caused the feed solution to be injected into the chip. Upon observation of the color of the product at the exit of the chip, after discarding the first 5 milky white droplets (about 100 µL), the follow-up sample was collected into an EP tube. The collected product was placed in a dialysis bag and dialyzed for 6 h (MWCO: 100 KDa) in 10 mM PBS (pH 7.4) intervals. Subsequently, it was stored at 4°C.

Tests were performed to calculate the encapsulation rate of the products according to the instructions of Ribogreen kit. Particle size and PDI detection, zeta potential analysis were performed on a Malvern Zetasizer nano instrument using standard assays.

The results of particle size, PDI and encapsulation rate of LNP loaded with mRNA prepared in this example are shown in Table 1.

**Table 1**

| No. | Cationic lipid | Lipid molar ratio Cationic: DSPC: cholesterol: PEG lipid | Encapsulation rate (%) | Particle size (nm) | PDI | Zeta potential (mV) |
|---|---|---|---|---|---|---|
| 1 | Compound 1 | 46.3: 9.4: 42.7: 1.6 | 87.8 | 97.3 | 0.118 | 0.485 |
| 2 | Compound 2 | 46.3: 9.4: 42.7: 1.6 | 90.1 | 100.3 | 0.224 | 0.233 |
| 3 | Compound 3 | 46.3: 9.4: 42.7: 1.6 | 94.8 | 86.4 | 0.126 | 0.254 |
| 4 | Compound 4 | 50: 10: 38.5: 1.5 | 92.3 | 115.2 | 0.232 | 0.401 |
| 5 | Compound 5 | 50: 10: 38.5: 1.5 | 86.3 | 106.3 | 0.125 | 0.206 |
| 6 | Compound 6 | 50: 10: 38.5: 1.5 | 88.9 | 95.6 | 0.119 | 0.215 |
| 7 | Compound 7 | 50: 10: 38.5: 1.5 | 90.2 | 99.7 | 0.109 | 0.335 |
| 8 | Compound 8 | 46.3: 9.4: 42.7: 1.6 | 88.6 | 100.7 | 0.094 | 0.319 |
| 9 | Compound 9 | 46.3: 9.4: 42.7: 1.6 | 78.9 | 105.3 | 0.129 | 0.109 |
| 10 | Compound 10 | 46.3: 9.4: 42.7: 1.6 | 89.3 | 99.8 | 0.288 | 0.118 |
| 11 | Compound 11 | 50: 10: 38.5: 1.5 | 88.2 | 108.3 | 0.365 | 0.293 |
| 12 | Compound 12 | 46.3: 9.4: 42.7: 1.6 | 91.8 | 122.4 | 0.194 | 0.321 |
| 13 | Compound 13 | 46.3: 9.4: 42.7: 1.6 | 84.7 | 107.7 | 0.201 | 0.382 |
| 14 | Compound 14 | 46.3: 9.4: 42.7: 1.6 | 90.3 | 90.1 | 0.211 | 0.463 |
| 15 | Compound 15 | 50: 10: 38.5: 1.5 | 91.1 | 111.5 | 0.099 | 0.195 |
| 16 | ALC-0315 | 46.3: 9.4: 42.7: 1.6 | 76.5 | 86.6 | 0.209 | 0.325 |
| 17 | MC3 | 50: 10: 38.5: 1.5 | 86.5 | 110.2 | 0.176 | 0.442 |
| 18 | Lipid 5 | 50: 10: 38.5: 1.5 | 92.3 | 105.3 | 0.263 | 0.329 |
| 19 | SM 102 | 50: 10: 38.5: 1.5 | 88.6 | 94.43 | 0.255 | 0.153 |

### Example 17 - Determination of the expression effect of delivering luciferase mRNA in vivo using nano-lipid particle compositions

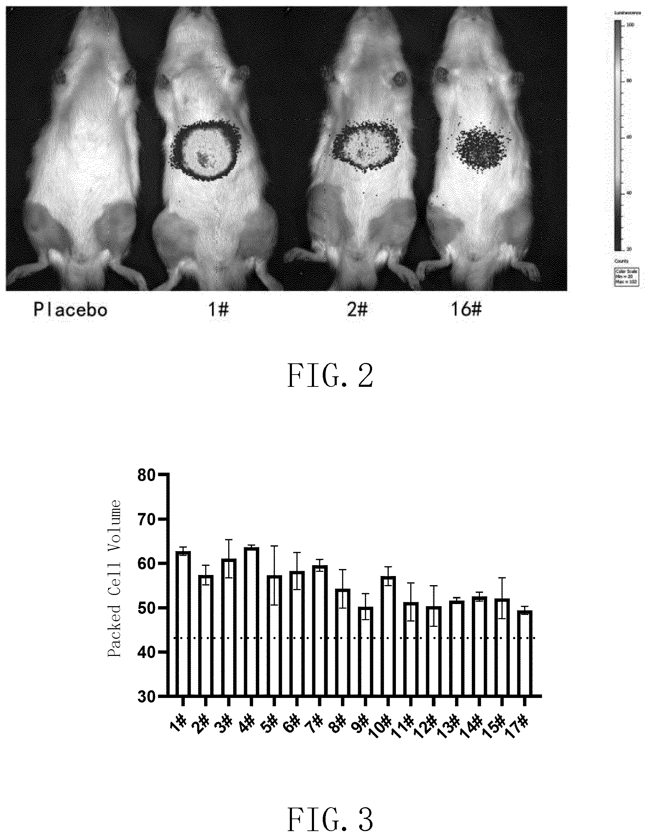

LUC-mRNA-lipid nanoparticles, prepared as in Example 16, containing 30 µg mRNA (see SEQ ID NO:1 for the corresponding nucleotide sequence of LUC-mRNA) were injected intramuscularly in 6-8 week old female SD rats. 200 µg of D-Luciferin Potassium Salt was injected into the tail vein of mice at specific time points and assayed using the PerkinElmer Small Animal Imaging System. Fluc is commonly used in mammalian cell cultures to measure gene expression and cell viability. It emits biological light in the presence of the substrate fluorescein. The basic characteristics of the mRNA used include ARCA cap structure, a polyA tail length of 100-120 nt, and complete substitution of pseudouridine. The results of the assay are shown in Fig. 2 and Table 2. Nano-lipid particle compositions comprising one of Compounds 1-15# delivered mRNA to the liver at levels superior to that of Compound 16# (ALC-0315).

**Table 2**

| No. | *In vivo* activity (30 µg) | Structure |
|---|---|---|
| 1 | 8.55E+07 | 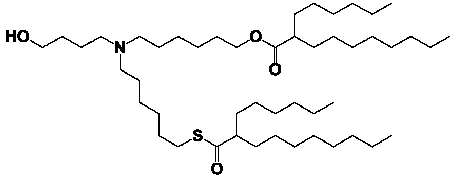 |
| 2 | 3.23E+07 | 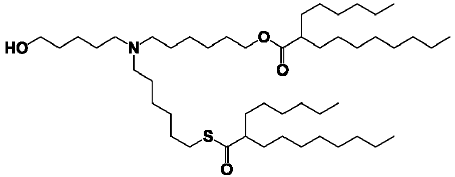 |
| 3 | 4.85E+07 | 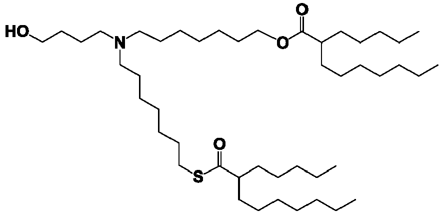 |
| 4 | 3.18E+07 | 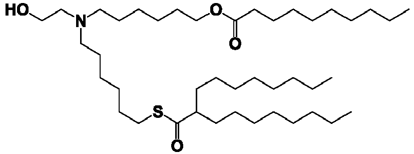 |
| 5 | 3.66E+07 | 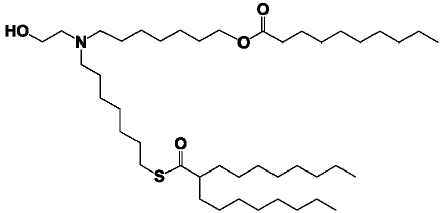 |
| 6 | 6.15E+07 | 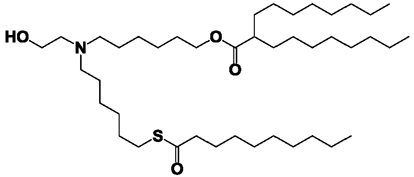 |
| 7 | 5.94E+07 | 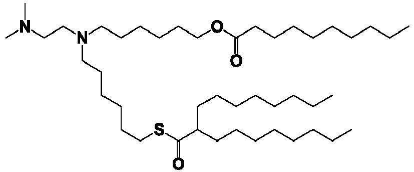 |
| 8 | 8.25E+07 | 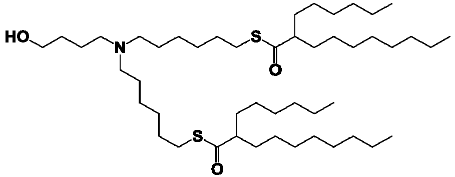 |
| 9 | 5.97E+07 | 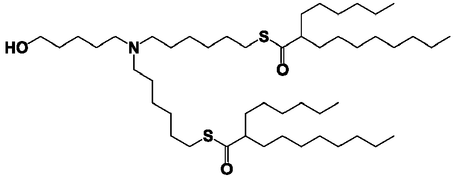 |
| 10 | 3.32E+07 | 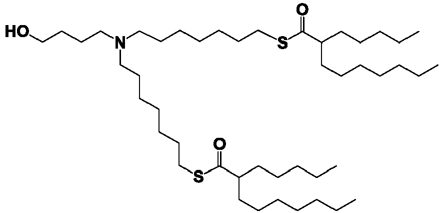 |
| 11 | 4.15E+07 | 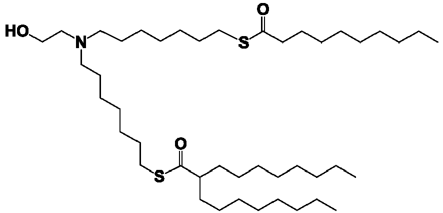 |
| 12 | 2.88E+07 | 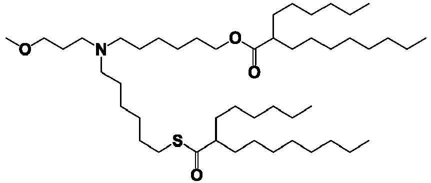 |
| 13 | 5.45E+07 | 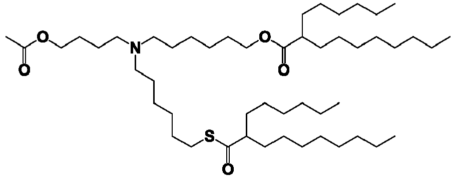 |
| 14 | 6.64E+07 | 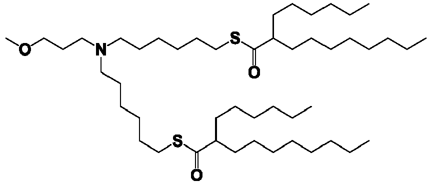 |
| 15 | 8.05E+07 | 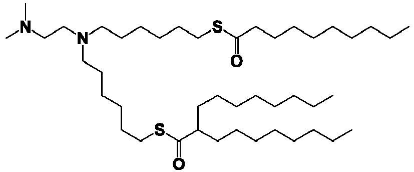 |
| 16 | 1.15E+07 | 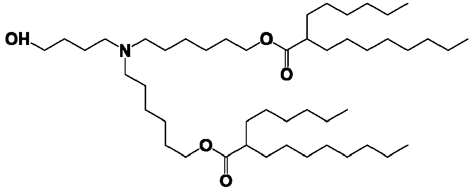 |

### Example 18 - Determination of the expression and effect of delivering erythropoietin (EPO) mRNA using nano-lipid particle compositions in mice

10 µg EPO-mRNA-lipid nanoparticles (see SEQ ID NO:2 for the corresponding nucleotide sequence of EPO-mRNA), prepared as in Example 16, were injected via tail vein injection in 6-8 week old female Balb/c mice. Animals were divided into 5 groups based on specific assay time points (0, 4, 12, 24, and 48 h). Eye frame blood sampling was performed on mice at specific time points and the packed cell volume was determined. hEPO is commonly used as a characterization gene for the level of protein expression in mammalian blood, which is proportional to the packed cell volume. The basic characteristics of the mRNA used include ARCA cap structure, a polyA tail length of 100-120 nt, and complete substitution of pseudouridine. The results of the assay are shown in Fig. 3. Seen from the results, the nano-lipid particle composition comprising Compound 1# delivered mRNA to the liver at a level superior to that of Compound 17# (MC3).

### Example 19 - Determination of the function of delivering monoclonal antibody Palivizumab mRNA using nano-lipid particle compositions in mice

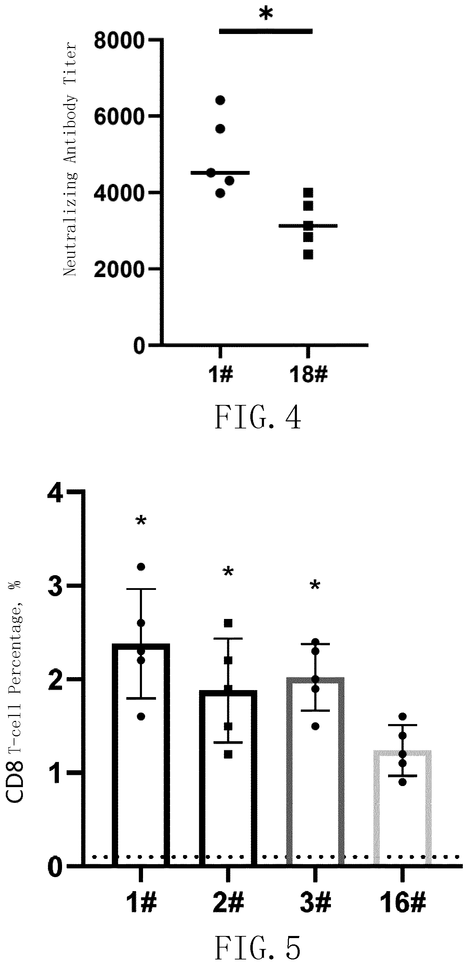

10 µg Palivizumab-mRNA-lipid nanoparticles (see SEQ ID NO:3 and SEQ ID NO:4 for the corresponding nucleotide sequence of Palivizumab-mRNA), prepared as in Example 16, were injected via intramuscular injection in 6-8 week old female Balb/c mice. Eye frame blood sampling was performed on mice at specific time points for 24 h, and the neutralizing antibody titer to RSV A2 in mouse serum was determined. HEp-2 cell culture was performed on 384-well microtitre plates. Petri dishes contained 15 ml of virus per well, with a tissue culture half-maximal infectious dose (TCID50) of 25-1000 and 15 ml of serially diluted serum per well, and were incubated for 1-1.5 h at 37°C and 5% CO₂. 2.5 × 10⁵ HEp-2 cells were added to each well and incubated at 37°C for 3-4 days. Cells were fixed with 80% acetone/20% phosphate-buffered saline for 15 min at 4°C under cryogenic conditions. Viral replication was detected using a horseradish peroxidase-conjugated anti-RSV F ELISA kit. Quantification was performed by monitoring absorbance at 450 nm by ELIASA, and IC₅₀ values were calculated using a nonlinear fitting algorithm in GraphPad Prism. The basic characteristics of the mRNA used inlucde ARCA cap structure, a polyA tail length of 100-120 nt, and complete substitution of pseudouridine. The results of the assay are shown in Fig. 4. The expression of Palivizumab-mRNA delivered by the cationic lipid prepared by Compound 1# was superior to that of Compound 18# (lipid 5), resulting in a better antiviral effect.

### Example 20 - Determination of the expression effect of delivering tumor antigen Kras-mRNA using nano-lipid particle compositions in mice

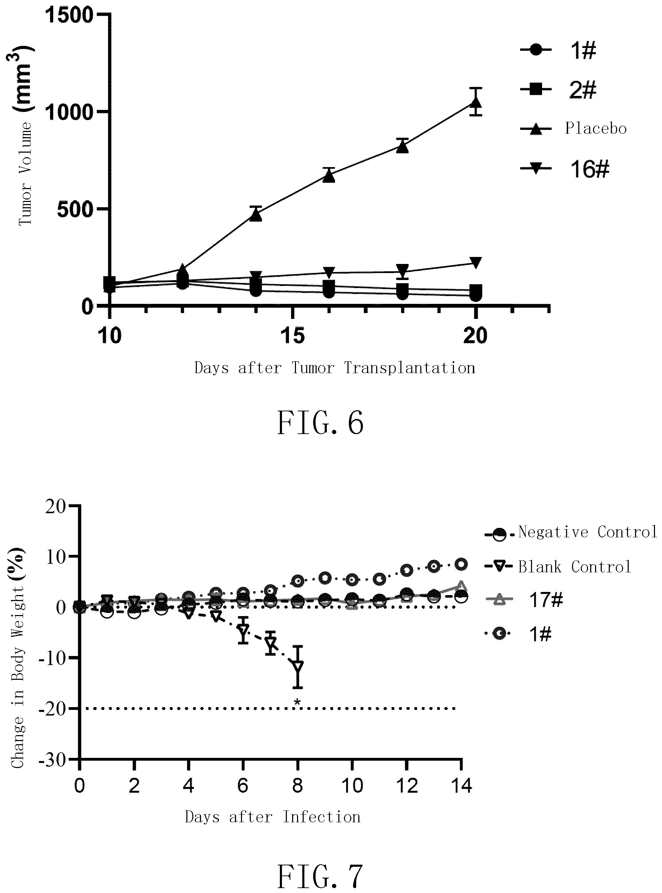

In 6-8 week old female NOG-SCID mice loaded with LU65, mice were injected with 10 µg G12C Kras-mRNA-lipid nanoparticles (see SEQ ID NO:5 for the nucleotide sequence corresponding to Kras-mRNA) , prepared as in Example 16, via intratumor injection once a week for 4 weeks. Eye frame blood sampling was performed on mice after 4 weeks. The antibody titer of anti-Kras G12C in the serum of the mice was determined, while detecting the cellular immunity level. The basic characteristics of the mRNA used include ARCA cap structure, a polyA tail length of 100-120 nt, and complete substitution of pseudouridine. The results of the assay are shown in Figs. 5 and 6. The Kras-mRNA delivered by the cationic lipids prepared by Compounds 1#, 2# and 3# induced cellular immunity superior to that of ALC-0315 (Fig. 5), resulting in a better tumor growth inhibition effect (Fig. 6).

### Example 21 - Delivery of novel coronavirus mRNA vaccines using nano-lipid particle composition

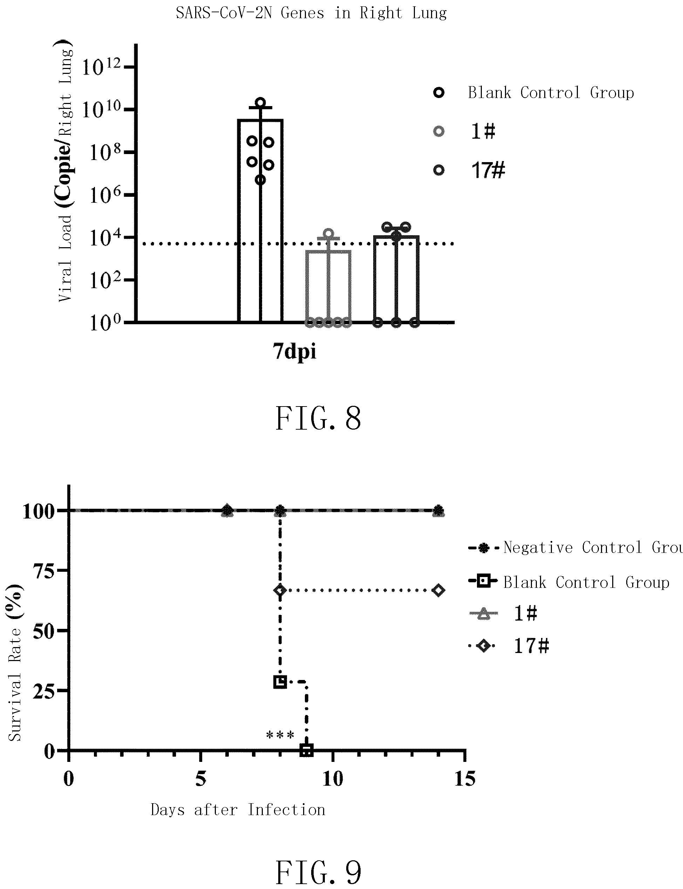

In a 6-week K18-hACE2 KI mouse model, immunization was performed by intramuscular injection of novel coronavirus pneumonia mRNA vaccine (see SEQ ID NO:6 for corresponding nucleotide sequence) delivered by different nano-lipid particle composition on days 0 and 14, and the animals were attacked on day 28 (day 14 after the final immunization) with a dose of 2.5 × 10³ PFU of novel coronavirus by nasal drip. The protective effect of novel coronavirus mRNA vaccines delivered by different nano-lipid particle compositions against infection with SARS-CoV-2 virus strains was evaluated. The basic characteristics of the mRNA used include ARCA cap structure, a polyA tail length of 100-120 nt, and complete substitution of pseudouridine. The results of the tests on body weight change, right lung viral load and survival rate after the attack are shown in Figs. 7, 8, and 9, respectively. Seen from the results, the nano-lipid particle composition comprising Compound 1# delivered mRNA at a level superior to that of Compound 17# (MC3).

### Example 22 - Delivery of respiratory syncytial virus mRNA vaccines using nano-lipid particle compositions

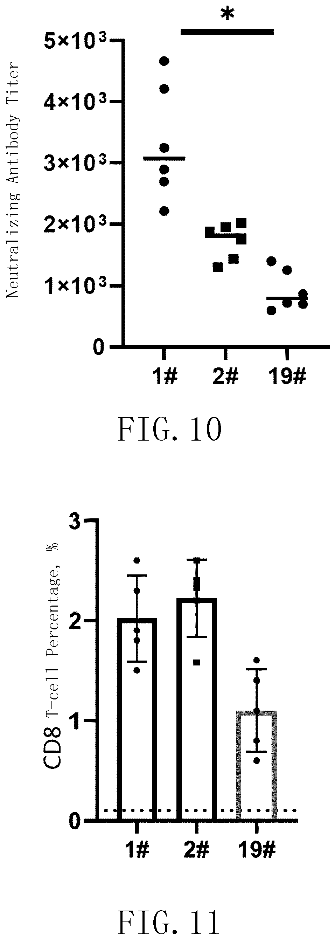

Female BALB/c mice from 5 to 7 weeks of age were immunized with novel coronavirus mRNA vaccines delivered by different nano-lipid particle compositions. The used mRNA encoded RSF F pre-fusion construct (DS-Cav1, see SEQ ID NO:7 of the corresponding nucleotide sequence) was immunized at 3-week intervals. Two weeks after the second immunization, blood was drawn for serological testing. Animals were executed 4 weeks after the second immunization and spleens were collected for ICS testing. The basic characteristics of the mRNA used inlcude ARCA cap structure, a polyA tail length of 100-120 nt, and complete substitution of pseudouridine. The neutralizing antibody titer and cellular immunity level in immunized mice are shown in Figs. 10 and 11. Seen from the results, the nano-lipid particle composition comprising Compound 1# delivered mRNA at a level superior to that of Compound 19# (SM102).

## Claims

1. A cationic lipid compound having a structure of formula (I): 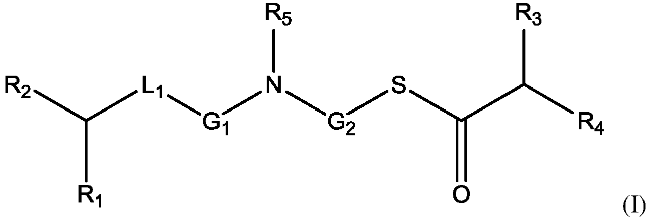
or pharmaceutically acceptable salts, tautomers or stereoisomers thereof, for delivery of nucleic acids,
wherein:
L₁ is -C(=O)S- or -C(=O)O-;
G₁ and G₂ are each independently a C₃-C₁₀ alkylene;
R₁ is a C₂-C₁₂ alkyl;
R₂ is H or a C₂-C₁₂ alkyl;
R₃ is a C₂-C₁₂ alkyl;
R₄ is H or a C₂-C₁₂ alkyl;
R₅ is H, a C₁-C₆ alkyl, R₇-OH, R₇-OC(=O)CH₃, R₇-NHC(=O)-CH₃, R₇-OCH₃ or R₇-N(R₈)₂;
R₇ is a C₂-C₁₈ alkylene;
R₈ is a straight or branched C₂-C₈ alkyl group, or forms a cyclic alkyl with N.

2. The cationic lipid compound according to claim 1, wherein G₁ and G₂ are C₄-C₈ alkylene.

3. The cationic lipid compound according to claim 1, wherein R₇ is a C₂-C₆ alkylene.

4. The cationic lipid compound according to claim 3, wherein R₅ is R₇-OH.

5. The cationic lipid compound according to claim 4, wherein R₁ and R₃ are each independently C₄-C₉ alkyl; and R₂ and R₄ are H or C₄-C₉ alkyl.

6. The cationic lipid compound according to claim 5, wherein only one of R₂ and R₄ is H.

7. The cationic lipid compound according to claim 1, wherein R₅ is R₇-N(CH₂CH₃)CH₂CH₃.

8. The cationic lipid compound according to claim 4, wherein the structure -C(R₁)R₂ or -C(R₃)R₄ in the structure of formula (I) each independently conforms to the following characteristics:

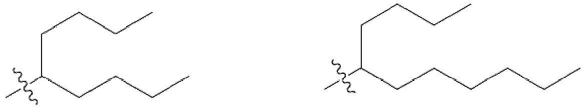

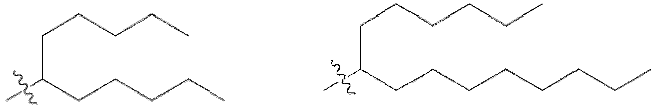

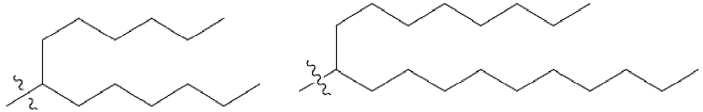

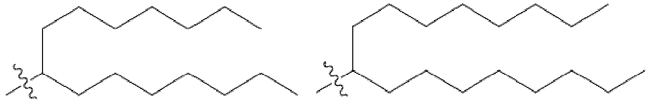

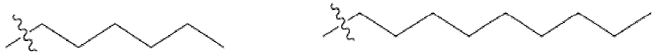

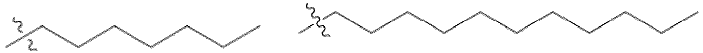

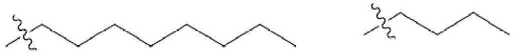

9. The cationic lipid compound according to claim 1, wherein the cationic lipid compound has one of the structures shown in the following table:
| No. | Structure |
|---|---|
| 1 | 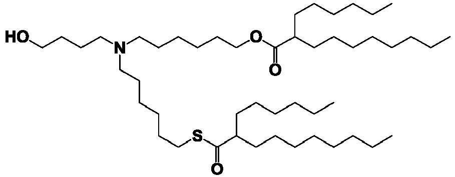 |
| 2 | 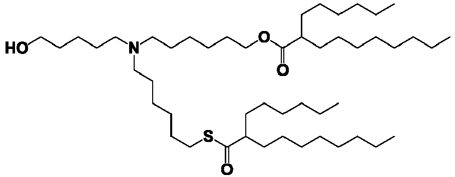 |
| 3 | 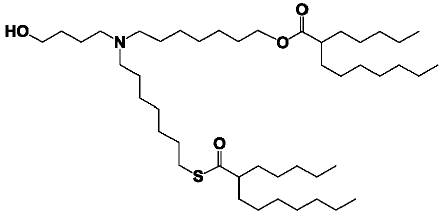 |
| 4 | 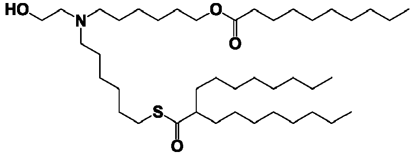 |
| 5 | 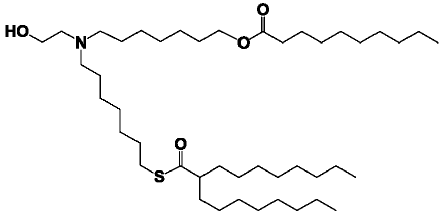 |
| 6 | 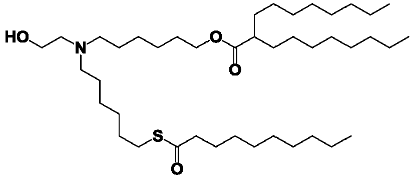 |
| 7 | 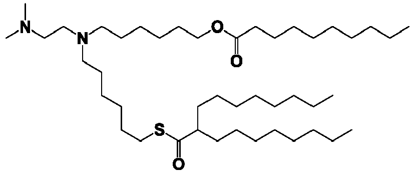 |
| 8 | 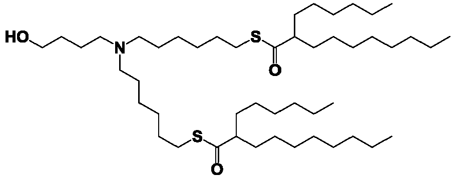 |
| 9 | 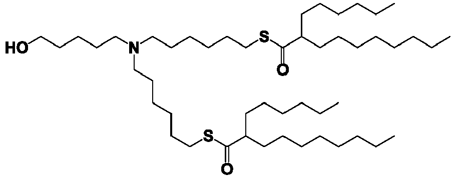 |
| 10 | 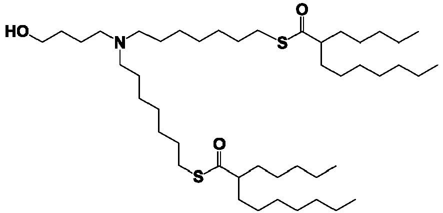 |
| 11 | 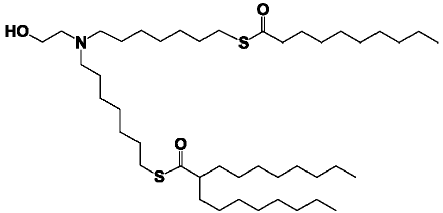 |
| 12 | 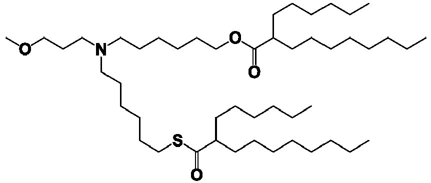 |
| 13 | 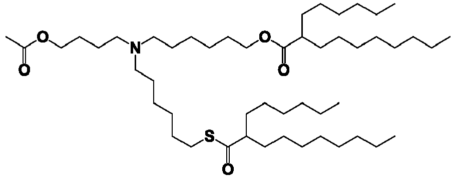 |
| 14 | 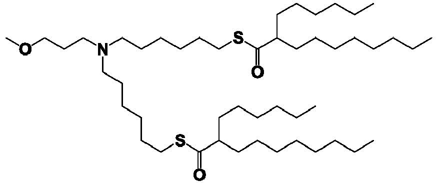 |
| 15 | 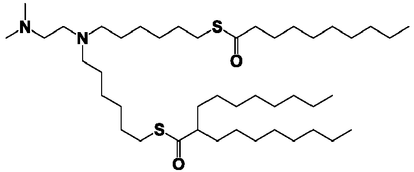 |
| 16 | 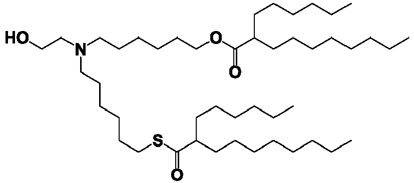 |
| 17 | 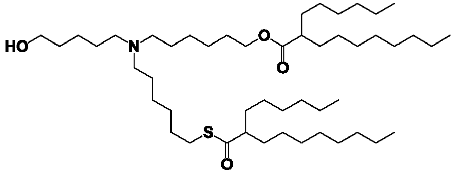 |
| 18 | 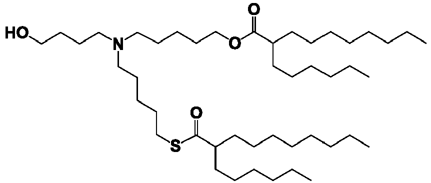 |
| 19 | 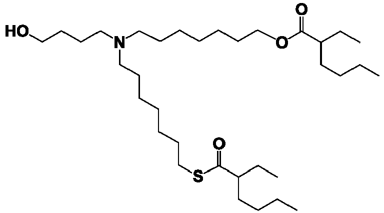 |
| 20 | 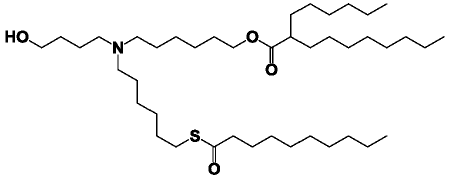 |
| 21 | 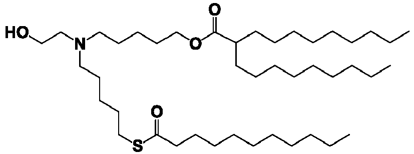 |
| 22 | 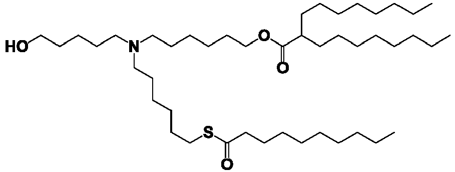 |
| 23 | 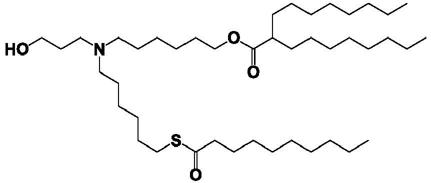 |
| 24 | 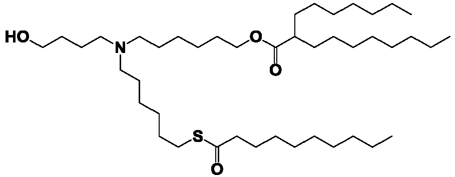 |
| 25 | 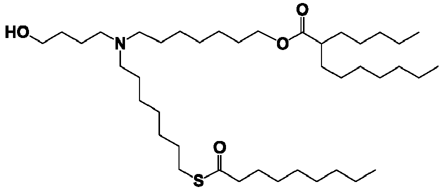 |
| 26 | 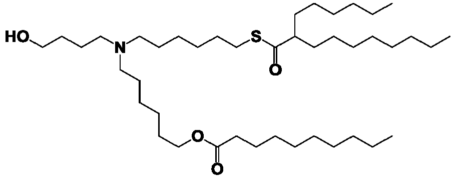 |
| 27 | 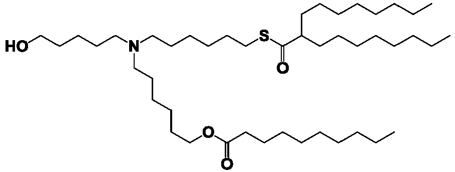 |
| 28 | 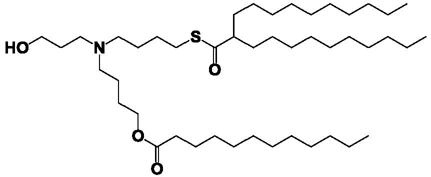 |
| 29 | 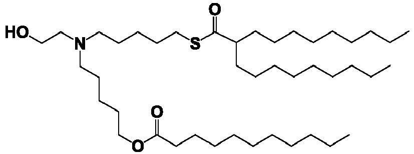 |
| 30 | 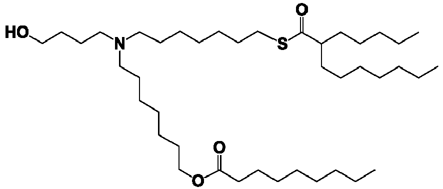 |
| 31 | 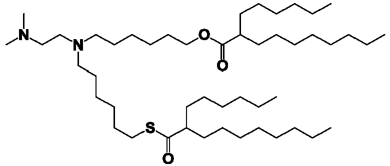 |
| 32 | 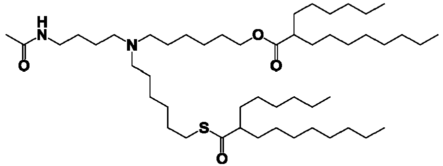 |
| 33 | 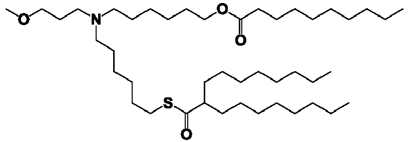 |
| 34 | 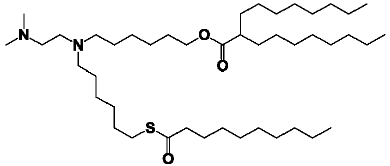 |
| 35 | 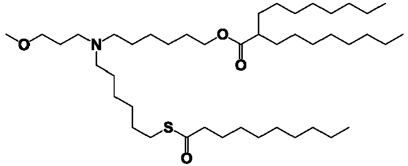 |
| 36 | 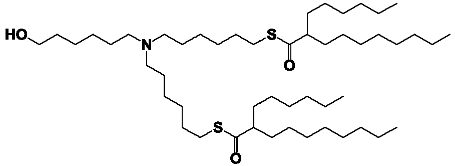 |
| 37 | 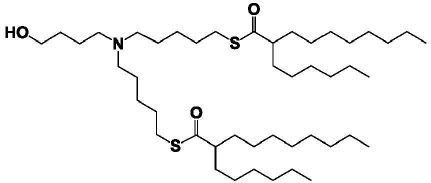 |
| 38 | 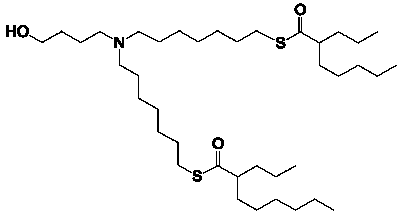 |
| 39 | 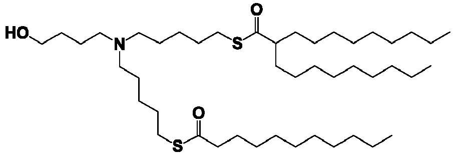 |
| 40 | 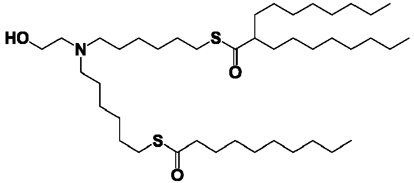 |
| 41 | 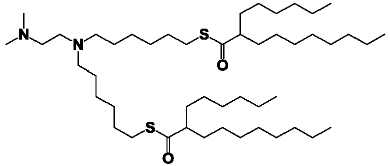 |
| 42 | 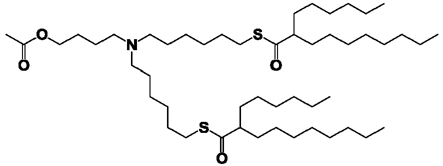 |
| 43 | 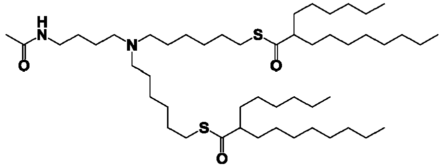 |
| 44 | 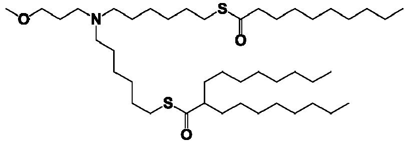 |
| 45 | 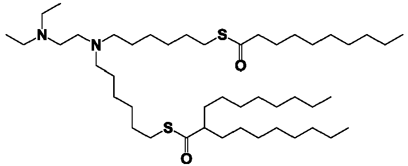 |

10. A liposomal formulation comprising the cationic lipid compound according to any one of claims 1-9 and a prophylactic or therapeutic nucleic acid, wherein the liposomal formulation is for use in the prevention or treatment of diseases.

11. The liposomal formulation according to claim 10, wherein the molar ratio of the nucleic acid to the cationic lipid compound is from 20:1 to 1:1.

12. The liposomal formulation according to claim 11, wherein the molar ratio of the nucleic acid to the cationic lipid compound is from 10:1 to 4:1.

13. The liposomal formulation according to claim 10, wherein the liposomal formulation has a diameter of 50 nm to 300 nm.

14. The liposomal formulation according to claim 13, wherein the liposomal formulation has a diameter of 50 nm to 150 nm, or 150 nm to 200 nm.

15. The liposomal formulation according to claim 10, further comprising one or more other lipid components including a neutral lipid, a steroid and a polymer-conjugated lipid.

16. The liposomal formulation according to claim 15, wherein the included steroid is cholesterol.

17. The liposomal formulation according to claim 16, wherein the molar ratio of the cholesterol to the cationic lipid compound is (0-1.5): 1.

18. The liposomal formulation according to claim 10, wherein the polymer in the polymer-conjugated lipid is polyethylene glycol (PEG).

19. The liposomal formulation according to claim 18, wherein the molar ratio of the cationic lipid compound to the polyethylene glycol-conjugated lipid is from 100:1 to 20:1.

20. The liposomal formulation according to claim 18, wherein the polyethylene glycol-conjugated lipid is PEG-DAG, PEG-PE, PEG-SDAG, PEG-cer, PEG-DMG or ALC-0159.

21. The liposomal formulation according to claim 15, wherein the neutral lipid is one or more selected from DSPC, DSPE, DPPC, DPPG, DMPC, DOPC, POPC, DOPE and SM.

22. The liposomal formulation according to claim 21, wherein the molar ratio of the cationic lipid compound to the neutral lipid is from 2:1 to 8:1.

23. The liposomal formulation according to claim 10, wherein the nucleic acid is selected from antisense RNA and/or messenger RNA.

24. Use of the cationic lipid compound according to any one of claims 1-9 or the liposomal formulation according to any one of claims 10-23 in the preparation of a medicament for inducing protein expression in a subject.

25. The use according to claim 24, wherein the subject is a mammal.

26. The use according to claim 24, wherein the subject is a non-human primate or human.
